# EUROPEAN PATENT APPLICATION

(11) **EP 2 894 163 A1**
(43) Date of publication of application: **15.07.2015**
(21) Application number: 12749806.1
(22) Date of filing: 08.02.2012
(51) Int. Cl.: C07K 14/605, C07K 19/00, C07K 1/06, C07K 1/04, A61K 38/26, A61P 3/10

(54) **A GLP-1 ANALOGUE, ITS PREPARATION METHODS AND USE THEREOF**

(30) Priority: 21.02.2011 CN 201110042039
(71) Applicant: Tianjin Institute Of Pharmaceutical Research, Nankai District, Tianjin 300193 (CN)
(72) Inventor: GONG, Min, Nankai District Tianjin 300193 (CN); FU, Gang, Nankai District Tianjin 300193 (CN); XU, Weiren, Nankai District Tianjin 300193 (CN); TANG, Lida, Nankai District Tianjin 300193 (CN); REN, Xiaowen, Nankai District Tianjin 300193 (CN); LIU, Peng, Nankai District Tianjin 300193 (CN); WANG, Yuli, Nankai District Tianjin 300193 (CN); WU, Jiang, Nankai District Tianjin 300193 (CN); ZOU, Meixiang, Nankai District Tianjin 300193 (CN)
(74) Representative: Coombes, Catherine Ann
(86) International application number: PCT/CN2012/070958
(87) International publication number: WO 2012/113286

(57) **Abstract**

Provided is a GLP-1 analogue having the structure as shown in the general formula A:

⁷HAEX₁₀TFTSX₁₅VSSYLEX₂₂QAAKEFIX₃₀WLX₃₃KGRG³⁷n₁X₁Cn₂X₂ (general 1 formula A),

wherein X₁₀ is glycine or cysteine, X₁₅ is aspartic acid or cysteine, X₂₂ is glycine or cysteine, X₃₀ is alanine or cysteine, X₃₃ is valine or cysteine, and at least one of X₁₀, X₁₅, X₂₂, X₃₀ and X₃₃ is cysteine, X₁ and X₂ respectively is glycine, alanine or valine, n₁=1-30, n₂=1-30. The general formula A contains two cysteines to form disulfide bonds. Also provided are the preparation methods and the use of said GLP-1 analogue. Said GLP-1 analogue has a prolonged blood half-life compared with GLP-1, and can be used for the treatment of diabetes and obesity.

## Description

### Technical Field

The present invention relates to the field of the medicaments associated with diabetes, specifically to a glucagon-like peptide-1 (GLP-1) analogue with a prolonged half-life of GLP-1 in vivo. The present invention also relates to a preparation method of the GLP-1 analogue and use of the GLP-1 analogue in the manufacture of a medicament for treating diabetes.

### Background Art

The Glucagon-like peptide-1 (hereinafter referred to as GLP-1) involved in the present invention is a polypeptide consisting of 37 amino acids mainly secreted by small intestinal L cells, and the active forms of GLP-1 are GLP-1(7-37)OH and GLP-1(7-36)NH2 (Mojsov S, J Clin Invest. 1987 Feb; 79(2): 616-9). GLP-1 can significantly reduce the blood glucose after meals in human, simulate the production of insulin, and meanwhile also play a role in reducing body weight without causing hypoglycemia (Drucker D J, Diabetes. 1998 Feb; 47(2): 159-69). Recent research also shows that GLP-1 has a pancreas regeneration effect (Drucker D J, 2003 Dec; 144(12): 5145-8). Moreover, GLP-1 is a fully humanized polypeptide, and thus possesses a great advantage in safety as a clinical drug. However, GLP-1(7-37) needs to be administered by injection for many times every day due to its serum half-life of only 3-5 minutes, and thus results in much inconvenience in the clinical use.

Recently, there are many researches using the GLP-1 analogue fusion protein technology to resolve the problem regarding the residence time of GLP-1 analogue in vivo (CN90101167.3, CN200710018734.2, CN200410054397.9, CN01820232.2, CN200380110152.7, CN200510039265.3, CN200610127237.1 and CN200910009642.7). However, the existing technologies are still far away from the ideal clinical goals, and generally fail to reach the clinical standard. Liraglutide, recently produced by Novo Norisk, is a GLP-1 analogue based on the modification of GLP-1 with palmitic acid, and has come into the market in America in 2009. However, Liraglutide also has the problem of a short half-life, and its dosage form still needs to be injected daily.

Therefore, there remains a need for a method of resolving the short half-life of GLP-1 in vivo.

### Disclosure of the Invention

Unless otherwise indicated, the "Fmoc strategy" as used herein refers to a synthetic method that amino acids with the amino terminal protected by Fmoc are condensed successively to synthesize a polypeptide in the presence of coupling reagent by using a polymer resin as the matrix of solid phase reaction. Specifically, please refer to Fmoc solid phase peptide synthesis: a practical approach, 2000, Oxford University Press.

In view of the defects that the clinically used GLP-1 analogues have short residence time in vivo and needs to be injected daily, one object of the present invention is to provide a GLP-1 analogue with longer half-life.

Another object of the present invention is to provide use of a GLP-1 analogue in the manufacture of a medicament for treating diabetes, and use of the GLP-1 analogue in the manufacture of a medicament for treating and/or preventing obesity.

Still another object of the present invention is to provide a pharmaceutical composition comprising the GLP-1 analogue described above as an active component, wherein the pharmaceutical composition further comprises one or more pharmaceutically acceptable auxiliary materials. Preferably, the pharmaceutical composition is an injection, further preferably is a lyophilized powder or a solution for injection.

The technical solutions for achieving the above objects are as follows:
In one aspect, the present invention provides a GLP-1 analogue having the following general formula A:

   ⁷HAEX₁₀T FTSX₁₅V SSYLE X₂₂QAAK EFIX₃₀W LX₃₃KGR G³⁷n₁X₁C n₂X₂ General Formula A,

   wherein X₁₀ is glycine or cysteine, X₁₅ is aspartic acid or cysteine, X₂₂ is glycine or cysteine, X₃₀ is alanine or cysteine, X₃₃ is valine or cysteine, and at least one of X₁₀, X₁₅, X₂₂, X₃₀ and X₃₃ is cysteine, n₁X₁ means that the number of X₁ is n₁, n₁=1-30, and X₁ is glycine, alanine or valine; n₂X₂ means that the number of X₂ is n₂, n₂=0-30, and X₂ is glycine, alanine or valine; and both of the two cysteines contained in the general formula A form disulfide bonds.

Preferably, the GLP-1 analogue has the following general formula I:

⁷HAEX₁₀T FTSX₁₅V SSYLE X₂₂QAAK EFIX₃₀W LX₃₃KGR G³⁷n₁X₁C n₂X₂ General Formula I,

wherein n₁=3-30, n₂=3-30.

The GLP-1 analogue having the general formula I such as: 
SEQ ID NO 82: ⁷HAEGT FTSCV SSYLE GQAAK EFIAW LVKGR G³⁷
GGGGG GGGGG C GGGGG GGGGG;
 
SEQ ID NO 83: ⁷HAEGT FTSDV SSYLE CQAAK EFIAW LVKGR G³⁷
GGGGG C GGGGG GGGGG;
 or

SEQ ID NO 84: 7HAEGT FTSDV SSYLE GQAAK EFICW LVKGR G37
GGG C GGGGG GGGGGGGGGGGGG.

Preferably, the GLP-1 analogue has the following general formula II:

⁷HAEGT FTSCV SSYLE GQAAK EFIAW LVKGR G³⁷n₁X₁C n₂X₂ General Formula II,

n₁=1-20, n₂=0-25, and wherein X₁ is glycine, alanine or valine, X₂ is glycine, alanine or valine;
Preferably, n₁=3-20, n₂=3-25;
Further preferably, said n₁=5-15, n₂=3-9;
Further preferably, the GLP-1 analogue is: 
SEQ ID NO 1: ⁷HAEGT FTSCV SSYLE GQAAK EFIAW LVKGR
G³⁷GGGGG C GGG;
 
SEQ ID NO 2: ⁷HAEGT FTSCV SSYLE GQAAK EFIAW LVKGR
G³⁷GGGGG GGGGGC GGG;
 
SEQ ID NO 3: 7HAEGT FTSCV SSYLE GQAAK EFIAW LVKGR
G37GGGGG GGGGGGGGGC GGG;
 
SEQ ID NO 4: ⁷HAEGT FTSCV SSYLE GQAAK EFIAW LVKGR
G³⁷GGGGG C GGGGG G;
 
SEQ ID NO 5: ^7HAEGT FTSCV SSYLE GQAAK EFIAW LVKGR
G^37GGGGG C GGGGG GGGG;
 
SEQ ID NO 6: ⁷HAEGT FTSCV SSYLE GQAAK EFIAW LVKGR
G³⁷GGGGG GGGGGC GGGGG G;
 SEQ ID NO 7: ⁷HAEGT FTSCV SSYLE GQAAK EFIAW LVKGR G³⁷GGGGG GGGGGC GGGGG GGGG; 
SEQ ID NO 8: ⁷HAEGT FTSCV SSYLE GQAAK EFIAW LVKGR
G³⁷GGGGG GGGGGGGGGC GGGGG G;
 
SEQ ID NO 9: ⁷HAEGT FTSCV SSYLE GQAAK EFIAW LVKGR
G³⁷GGGGG GGGGGGGGGC GGGGG GGGG;
 
SEQ ID NO 10: ⁷HAEGT FTSCV SSYLE GQAAK EFIAW LVKGR
G³⁷AAAAA C AAA;
 
SEQ ID NO 11: ^7HAEGT FTSCV SSYLE GQAAK EFIAW LVKGR
G^37AAAAA AAAAAC AAA;
 
SEQ ID NO 12: ⁷HAEGT FTSCV SSYLE GQAAK EFIAW LVKGR
G³⁷AAAAA AAAAAAAAAAC AAA;
 
SEQ ID NO 13: ⁷HAEGT FTSCV SSYLE GQAAK EFIAW LVKGR
G³⁷AAAAA C AAAAA A;
 
SEQ ID NO 14: ⁷HAEGT FTSCV SSYLE GQAAK EFIAW LVKGR
G³⁷AAAAA C AAAAA AAAA;
 
SEQ ID NO 15: ^7HAEGT FTSCV SSYLE GQAAK EFIAW LVKGR
G^37AAAAA AAAAAC AAAAA A;
 
SEQ ID NO 16: ⁷HAEGT FTSCV SSYLE GQAAK EFIAW LVKGR
G³⁷AAAAA AAAAAC AAAAA AAAA;
 
SEQ ID NO 17: ⁷HAEGT FTSCV SSYLE GQAAK EFIAW LVKGR
G³⁷AAAAA AAAAAAAAAAC AAAAA A;
 
SEQ ID NO 18: ⁷HAEGT FTSCV SSYLE GQAAK EFIAW LVKGR
G³⁷AAAAA AAAAAAAAAAAAAAC AAAAA AAAA;
 
SEQ ID NO 19: ⁷HAEGT FTSCV SSYLE GQAAK EFIAW LVKGR
G³⁷VVVVV C VVV;
 
SEQ ID NO 20: ⁷HAEGT FTSCV SSYLE GQAAK EFIAW LVKGR
G³⁷VVVVV VVVVVC VVV;
 
SEQ ID NO 21: ⁷HAEGT FTSCV SSYLE GQAAK EFIAW LVKGR
G³⁷VVVVV VVVVVVVVVVC VVV;
 
SEQ ID NO 22: ⁷HAEGT FTSCV SSYLE GQAAK EFIAW LVKGR
G³⁷VVVVV C VVVVV V;
 
SEQ ID NO 23: ⁷HAEGT FTSCV SSYLE GQAAK EFIAW LVKGR
G³⁷VVVVV C VVVVV VVVV;
 
SEQ ID NO 24: ⁷HAEGT FTSCV SSYLE GQAAK EFIAW LVKGR
G³⁷VVVVV VVVVVC VVVVV V;
 
SEQ ID NO 25: ⁷HAEGT FTSCV SSYLE GQAAK EFIAW LVKGR
G³⁷VVVVV VVVVVC VVVVV VVVV;
 
SEQ ID NO 26: ⁷HAEGT FTSCV SSYLE GQAAK EFIAW LVKGR
G³⁷VVVVV VVVVVVVVVVC VVVVV V;
 or

SEQ ID NO 27: ⁷HAEGT FTSCV SSYLE GQAAK EFIAW LVKGR
G³⁷VVVVVV VVVVVVVVVVVC VVVVV VVVV.

Furthermore, the GLP-1 analogue having general formula A may also have the following general formula III:

⁷HAEGT FTSDV SSYLE GQAAK EFICW LVKGR G³⁷ n₁X₁ C n₂X₂ General Formula III,

wherein, n₁=1-10, n₂=0-25, X₁ is glycine, alanine or valine, X₂ is glycine, alanine or valine;
Preferably, n₁=3-10, n₂=3-25;
Or n₁=1-5, n₂=10-20;
More preferably, n₁=3-5, n₂=10-20.

Preferably, the GLP-1 analogue is: 
SEQ ID NO 28: ⁷HAEGT FTSDV SSYLE GQAAK EFICW LVKGR G³⁷G
C GGGGG GGGGG;
 
SEQ ID NO 29: ⁷HAEGT FTSDV SSYLE GQAAK EFICW LVKGR G³⁷G
C GGGGG GGGGGGGGG;
 
SEQ ID NO 30: ⁷HAEGT FTSDV SSYLE GQAAK EFICW LVKGR G³⁷G
C GGGGG GGGGGGGGGGGGGGGGGGGGGGG;
 
SEQ ID NO 31: ⁷HAEGT FTSDV SSYLE GQAAK EFICW LVKGR
G³⁷GG C GGGGG GGGGG;
 
SEQ ID NO 32: ⁷HAEGT FTSDV SSYLE GQAAK EFICW LVKGR
G³⁷GG C GGGGG GGGGGGGGG;
 
SEQ ID NO 33: ⁷HAEGT FTSDV SSYLE GQAAK EFICW LVKGR
G³⁷GG C GGGGG GGGGGGGGGGGGG;
 
SEQ ID NO 34: ⁷HAEGT FTSDV SSYLE GQAAK EFICW LVKGR
G³⁷GGGGG C GGGGG GGGGG;
 SEQ ID NO 35: ⁷HAEGT FTSDV SSYLE GQAAK EFICW LVKGR G³⁷GGGGG C GGGGG GGGGGGGGGG; 
SEQ ID NO 36: ⁷HAEGT FTSDV SSYLE GQAAK EFICW LVKGR
G³⁷GGGGG C GGGGG GGGGGGGGGGGGG;
 
SEQ ID NO 37: ⁷HAEGT FTSDV SSYLE GQAAK EFICW LVKGR G³⁷A
C AAAAA AAAAA;
 
SEQ ID NO 38: ⁷HAEGT FTSDV SSYLE GQAAK EFICW LVKGR G³⁷A
C AAAAA AAAAAAAAAA;
 
SEQ ID NO 39: ⁷HAEGT FTSDV SSYLE GQAAK EFICW LVKGR G³⁷A
C AAAAA AAAAAAAAAAAAAAAAAAA;
 
SEQ ID NO 40: ^7HAEGT FTSDV SSYLE GQAAK EFICW LVKGR
G^37AA C AAAAA AAAAA;
 
SEQ ID NO 41: ⁷HAEGT FTSDV SSYLE GQAAK EFICW LVKGR
G³⁷AA C AAAAA AAAAAAAAAA;
 
SEQ ID NO 42: ⁷HAEGT FTSDV SSYLE GQAAK EFICW LVKGR
G³⁷AA C AAAAA AAAAAAAAAAAAAAAA;
 
SEQ ID NO 43: ⁷HAEGT FTSDV SSYLE GQAAK EFICW LVKGR
G³⁷AAAAA C AAAAA AAAAA;
 
SEQ ID NO 44: ⁷HAEGT FTSDV SSYLE GQAAK EFICW LVKGR
G³⁷AAAAA C AAAAA AAAAAAAAAA;
 
SEQ ID NO 45: ⁷HAEGT FTSDV SSYLE GQAAK EFICW LVKGR
G³⁷AAAAA C AAAAA AAAAAAAAAAAAAAAA;
 
SEQ ID NO 46: ⁷HAEGT FTSDV SSYLE GQAAK EFICW LVKGR G³⁷V
C VVVVVV VVVVVV;
 
SEQ ID NO 47: ⁷HAEGT FTSDV SSYLE GQAAK EFICW LVKGR G³⁷V
C VVVVVV VVVVVVVVVVV;
 
⁷HAEGT FTSDV SSYLE GQAAK EFICW LVKGR G³⁷V
C VVVVVV VVVVVVVVVVVVVVVV;
 
SEQ ID NO 49: ⁷HAEGT FTSDV SSYLE GQAAK EFICW LVKGR
G³⁷VV C VVVVV VVVVV;
 
SEQ ID NO 50: ⁷HAEGT FTSDV SSYLE GQAAK EFICW LVKGR
G³⁷VV C VVVVV VVVVVVVVVV;
 
SEQ ID NO 51: ⁷HAEGT FTSDV SSYLE GQAAK EFICW LVKGR
G³⁷VV C VVVVVV VVVVVVVVVVVVVVVVV;
 
SEQ ID NO 52: ⁷HAEGT FTSDV SSYLE GQAAK EFICW LVKGR
G³⁷VVVVV C VVVVV VVVVV;
 
SEQ ID NO 53: ⁷HAEGT FTSDV SSYLE GQAAK EFICW LVKGR
G³⁷VVVVVV C VVVVVV VVVVVVVVVVV;
 or

SEQ ID NO 54: ⁷HAEGT FTSDV SSYLE GQAAK EFICW LVKGR
G³⁷VVVVVV C VVVVVV VVVVVVVVVVVVVV.

Furthermore, the GLP-1 analogue having general formula A may also have the following general formula IV:

⁷HAEGT FTSDV SSYLE GQAAK EFICW LCKGR G³⁷ n₁X₁ C n₂X₂ General Formula IV,

wherein n₁=1-10, n₂=0-30, X₁ is glycine, alanine or valine, X₂ is glycine, alanine or valine;
Preferably, n₁=3-10, n₂=3-30;
Or n₁=1-5, n₂=10-20;
More preferably, n₁=3-5, n₂=10-20.

Preferably, the GLP-1 analogue is: 
SEQ ID NO 55: ⁷HAEGT FTSDV SSYLE GQAAK EFICW LCKGR G³⁷G
C GGGGG GGGGG;
 
SEQ ID NO 56: ⁷HAEGT FTSDV SSYLE GQAAK EFICW LCKGR G³⁷G
C GGGGG GGGGGGGGGG;
 
SEQ ID NO 57: ⁷HAEGT FTSDV SSYLE GQAAK EFICW LCKGR G³⁷G
C GGGGG GGGGGGGGGGGGGGG;
 
SEQ ID NO 58: ⁷HAEGT FTSDV SSYLE GQAAK EFICW LCKGR
G³⁷GG C GGGGG GGGGG;
 
SEQ ID NO 59: ⁷HAEGT FTSDV SSYLE GQAAK EFICW LCKGR
G³⁷GG C GGGGG GGGGGGGGGG;
 
SEQ ID NO 60: ⁷HAEGT FTSDV SSYLE GQAAK EFICW LCKGR
G³⁷GG C GGGGG GGGGGGGGGGGGG;
 
SEQ ID NO 61: ⁷HAEGT FTSDV SSYLE GQAAK EFICW LCKGR
G³⁷GGGGG C GGGGG GGGGG;
 
SEQ ID NO 62: ⁷HAEGT FTSDV SSYLE GQAAK EFICW LCKGR
G³⁷GGGGG C GGGGG GGGGGGGGG;
 SEQ ID NO 63: ⁷HAEGT FTSDV SSYLE GQAAK EFICW LCKGR G37GGGGG C GGGGG GGGGGGGGGGGGGGG; 
SEQ ID NO 64: ⁷HAEGT FTSDV SSYLE GQAAK EFICW LCKGR G³⁷A
C AAAAA AAAAA;
 
SEQ ID NO 65: ⁷HAEGT FTSDV SSYLE GQAAK EFICW LCKGR G³⁷A
C AAAAA AAAAAAAAAA;
 
SEQ ID NO 66: 7HAEGT FTSDV SSYLE GQAAK EFICW LCKGR G37A
C AAAAA AAAAAAAAAAAAAAAAA;
 
SEQ ID NO 67: ⁷HAEGT FTSDV SSYLE GQAAK EFICW LCKGR
G³⁷AA C AAAAA AAAAA;
 
SEQ ID NO 68: ⁷HAEGT FTSDV SSYLE GQAAK EFICW LCKGR
G³⁷AA C AAAAA AAAAAAAAAA;
 
SEQ ID NO 69: ⁷HAEGT FTSDV SSYLE GQAAK EFICW LCKGR
G³⁷AA C AAAAA AAAAAAAAAAAAAAAAA;
 
SEQ ID NO 70: ⁷HAEGT FTSDV SSYLE GQAAK EFICW LCKGR
G³⁷AAAAA C AAAAA AAAAA;
 
SEQ ID NO 71: ⁷HAEGT FTSDV SSYLE GQAAK EFICW LCKGR
G³⁷AAAAA C AAAAA AAAAAAAAAA;
 
SEQ ID NO 72: ⁷HAEGT FTSDV SSYLE GQAAK EFICW LCKGR
G³⁷AAAAA C AAAAA AAAAAAAAAAAAAAAAAAAA;
 
SEQ ID NO 73: ⁷HAEGT FTSDV SSYLE GQAAK EFICW LCKGR G³⁷V
C VVVVV VVVVV;
 
SEQ ID NO 74: ⁷HAEGT FTSDV SSYLE GQAAK EFICW LCKGR G³⁷V
C VVVVVV VVVVVVVVVVV;
 
⁷HAEGT FTSDV SSYLE GQAAK EFICW LCKGR G³⁷V
C VVVVVV VVVVVVVVVVVVVVVVV;
 
SEQ ID NO 76: ⁷HAEGT FTSDV SSYLE GQAAK EFICW LCKGR
G³⁷VV C VVVVV VVVVV;
 
SEQ ID NO 77: ⁷HAEGT FTSDV SSYLE GQAAK EFICW LCKGR
G³⁷VV C VVVVV VVVVVVVVVV;
 
SEQ ID NO 78: ⁷HAEGT FTSDV SSYLE GQAAK EFICW LCKGR
G³⁷VV C VVVVV VVVVVVVVVVVVVVVV;
 
SEQ ID NO 79: ⁷HAEGT FTSDV SSYLE GQAAK EFICW LCKGR
G³⁷VVVVV C VVVVV VVVVV;
 
SEQ ID NO 80: ⁷HAEGT FTSDV SSYLE GQAAK EFICW LCKGR
G³⁷VVVVVV C VVVVVV VVVVVVVVVVV;
 or

SEQ ID NO 81: ⁷HAEGT FTSDV SSYLE GQAAK EFICW LCKGR
G³⁷VVVVV C VVVVV VVVVVVVVVVVVVVVV.

In another aspect, the present invention provides a preparation method of the GLP-1 analogue described above comprising solid phase polypeptide synthesis in accordance with Fmoc strategy.

In still another aspect, the present invention provides use of the GLP-1 analogue described above in the manufacture of a medicament for treating diabetes, obesity, diseases associated with diabetes and obesity associated with diabetes as well as for preventing obesity.

Moreover, the present invention provides a pharmaceutical composition comprising the GLP-1 analogue as claimed in any one of claims 1-8 and one or more pharmaceutically acceptable auxiliary materials.

Preferably, the pharmaceutical composition is an injection, further preferably is a lyophilized powder or a solution for injection.

Below is the detailed description of the present invention:

### (1) GLP-1 analogue

The general formula of the GLP-1 analogue described in the present invention is as follows:

General formula A, ⁷HAEX₁₀T FTSX₁₅V SSYLE X₂₂QAAK EFIX₃₀W LX₃₃KGR G³⁷n₁X₁C n₂X₂ ⁷HAEGT FTSDV SSYLE GQAAK EFIAW LVKGR G³⁷

is a humanized GLP-1 sequence. The GLP-1 analogue of the above general formula A is an artificial sequence, in which the amino acid at position 10, 15, 22, 30 or 33 is modified by replacing the original amino acid with cysteine respectively. Furthermore, in the above general formula of amino acid, X₁ may be Gly, Ala or Val; and X₂ may be Gly, Ala or Val. At last, n₁ and n₂ in the general formula refer to that the number of the hydrophobic amino acid (Gly, Ala or Val) at C terminal of the polypeptide are n₁ and n₂, n₁=1-30, n₂=0-30. In the above general formula of polypeptide, both of the two cysteines form disulfide bonds.

Preferably, the general formula of GLP-1 analogue is as follows:

⁷HAEX₁₀T FTSX₁₅V SSYLE X₂₂QAAK EFIX₃₀W LX₃₃KGR G³⁷n₁X₁C n₂X₂ General Formula I,

wherein n₁=3-30, n₂=3-30.

Preferably, the general formula of the mutated amino acid sequences having cysteines at positions 15, 30 and 33 are as follows:

General formula II: ⁷HAEGT FTSCV SSYLE GQAAK EFIAW LVKGR G³¹n₁X₁C n₂X₂, n₁=1-20; n₂=0-25; preferably, n₁=3-20, n₂=3-25; wherein X₁ is Gly, Ala or Val; X₂ is Gly, Ala or Val.

General formula III: ⁷HAEGT FTSDV SSYLE GQAAK EFICW LVKGR G³⁷ n₁X₁ C n₂X₂, n₁=1-10, n₂=0-25; preferably, n₁=3-10, n₂=3-25; wherein X₁ is Gly, Ala or Val; X₂ is Gly, Ala or Val.

General formula IV: ⁷HAEGT FTSDV SSYLE GQAAK EFICW LCKGR G³⁷ n₁X₁ C n₂X₂, n₁=1-10, n₂=0-30; preferably, n₁=3-10, n₂=3-30; wherein X₁ is Gly, Ala or Val; X₂ is Gly, Ala or Val.

The above general formula II of the polypeptide may be further optimized as:
⁷HAEGT FTSCV SSYLE GQAAK EFIAW LVKGR G³⁷ n₁X₁ C n₂X₂, n₁=5-15, n₂=3-9, wherein X₁ is Gly, Ala or Val; X₂ is Gly, Ala or Val.

The above general formula III of the polypeptide may be optimized as:
⁷HAEGT FTSDV SSYLE GQAAK EFICW LVKGR G³⁷ n₁X₁ C n₂X₂, n₁=1-5, n₂=10-20; preferably, n₁=3-5, n₂=10-20; wherein X₁ is Gly, Ala or Val; X₂ is Gly, Ala or Val.

The above general formula IV of the polypeptide may be optimized as:
⁷HAEGT FTSDV SSYLE GQAAK EFICW LCKGR G³⁷ n₁X₁ C n₂X₂, n₁=1-5, n₂=10-20; preferably, n₁=3-5, n₂=10-20; wherein X₁ is Gly, Ala or Val; X₂ is Gly, Ala or Val.

### (2) Pharmaceutical composition of the present invention

The GLP-1 analogue of the present invention can be prepared into a pharmaceutical composition in combination with one or more pharmaceutically acceptable auxiliary materials including water soluble filler, pH regulator, stabilizer, water for injection, osmotic pressure regulator and so on.

The water soluble filler of the present invention is one or more selected from the group consisting of mannitol, low molecular weight dextran, sorbitol, polyethylene glycol, glucose, lactose, galactose, and so on.

The pH regulator is one or more selected from the group consisting of non-volatile acids, such as citric acid, phosphoric acid, lactic acid, tartaric acid, hydrochloric acid and etc.; and physiologically acceptable organic or inorganic acids, bases and/or salts and so on, such as potassium hydroxide, sodium hydroxide or potassium hydroxide or ammonium hydroxide, sodium carbonate or potassium carbonate or ammonium carbonate, sodium bicarbonate or potassium bicarbonate or ammonium bicarbonate, and the like.

The stabilizer is one or more selected from the group consisting of EDTA-2Na, sodium thiosulfate, sodium metabisulfite, sodium sulfite, dipotassium hydrogen phosphate, sodium bicarbonate, sodium carbonate, arginine, glutamic acid, polyethylene glycol 6000, polyethylene glycol 4000, sodium dodecyl sulfate or trihydroxymethyl aminomethane and so on. The sodium metabisulfite, dipotassium hydrogen phosphate, arginine, polyethylene glycol 6000 and trihydroxymethyl aminomethane are preferred.

The osmotic pressure regulator is one or the combination of sodium chloride and potassium chloride.

### (3) Preparation method of injection

The pharmaceutical composition of the present invention can be administered by intramuscular, intravenous, or subcutaneous injection, and the preferable dosage form is a lyophilized powder or a solution for injection.

### The preparation method of the freeze-drying injection

To an appropriate amount of GLP-1 analogue solution, water soluble filler, stabilizer, osmotic pressure regulator and the like are added, and an appropriate amount of water for injection is added. The pH value is adjusted to 4-8 so as to dissolve the materials therein. The resulting solution is diluted to a proper concentration by adding water. 0.1-0.5% of active carbon is added to the solution, and then removed after the solution is stirred for at 0 - 10 °C for 10-20 minutes. The resulting solution is filtered with microfiltration membrane to remove bacteria. The filtrate is subpackaged, and then fabricated in accordance with freeze-drying method as a white, loose and blocky substance, which is sealed to obtain the freeze-drying injection. Each specification contains the GLP-1 analogue between 5µg and 1mg.

### Preparation method of the solution for injection

To an appropriate amount of GLP-1 analogue solution or freeze-dried powder, water soluble filler, stabilizer, osmotic pressure regulator and the like are added, and an appropriate amount of water for injection is added. The pH value is adjusted to 4-8 so as to dissolve the materials therein. The resulting solution is diluted to a proper concentration by adding water. 0.1-0.5% of active carbon is added the solution, and then removed after the solution is stirred at 0 - 10 °C for 10-20 minutes. The resulting solution is filtered with microfiltration membrane to remove bacteria. The filtrate is subpackaged, and sealed to obtain the solution for injection. Each specification contains the GLP-1 analogue between 5µg and 1mg.

### (4) Use of pharmaceutical composition

The GLP-1 analogue of the present invention can be used in the manufacture of a medicament for treating diabetes. Specifically, the composition of the present invention can be administered in the form of intravenous, intramuscular, or subcutaneous injection. Although the dosage is varied depending on the subject to be treated, mode of administration, symptoms and other factors, the GLP-1 analogue of the present invention is effective in a wide range of doses. In the treatment of the adults, the dosage range is between 5µg/person and 1mg/person, administered once daily or every several days. The actual dosage should be determined by a physician according to related conditions including the physical state of the patient to be treated, administration route, age, weight, individual response to the drug, severity of the patients' symptoms and the like. Therefore, the above dosage range does not limit the scope of the present invention in any way.

The GLP-1 analogue of the present invention has overcome the problem of the short half-life of GLP-1. The half-life of the GLP-1 analogue provided can reach above 15-75 hours in vivo, which is significantly longer than that of GLP-1 administered alone (with a half-life of only 3-5 minutes), thereby are greatly favorable for the clinical spreading and application of the GLP-1 analogue of the present invention.

### Brief Description of the Drawings

The examples of the present invention are illustrated in detail below with reference to the drawings, in which:

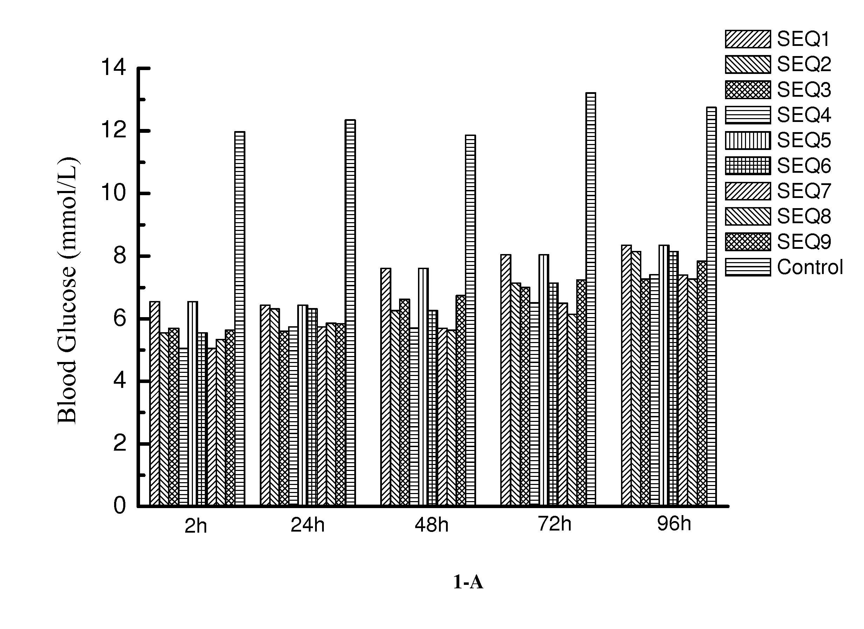

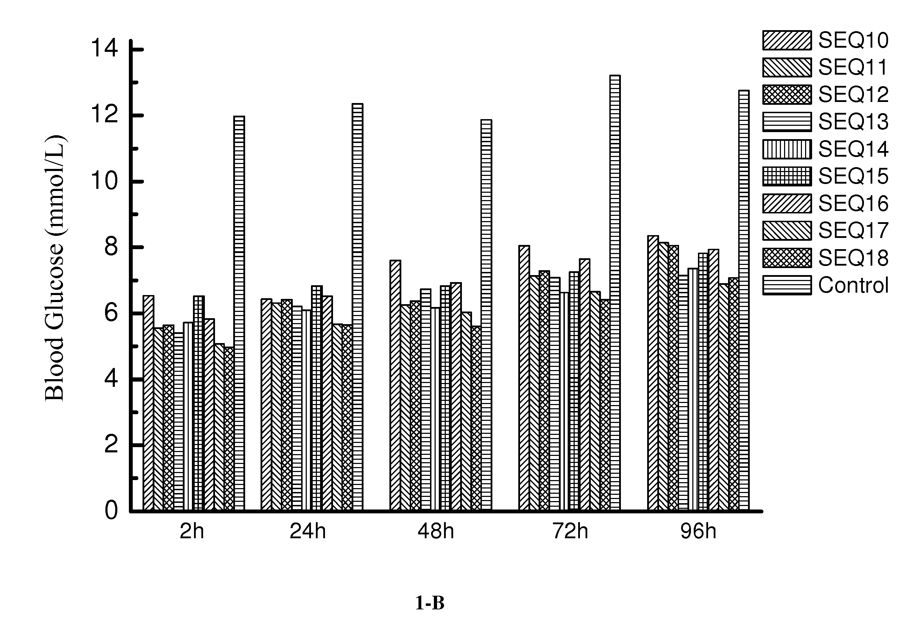

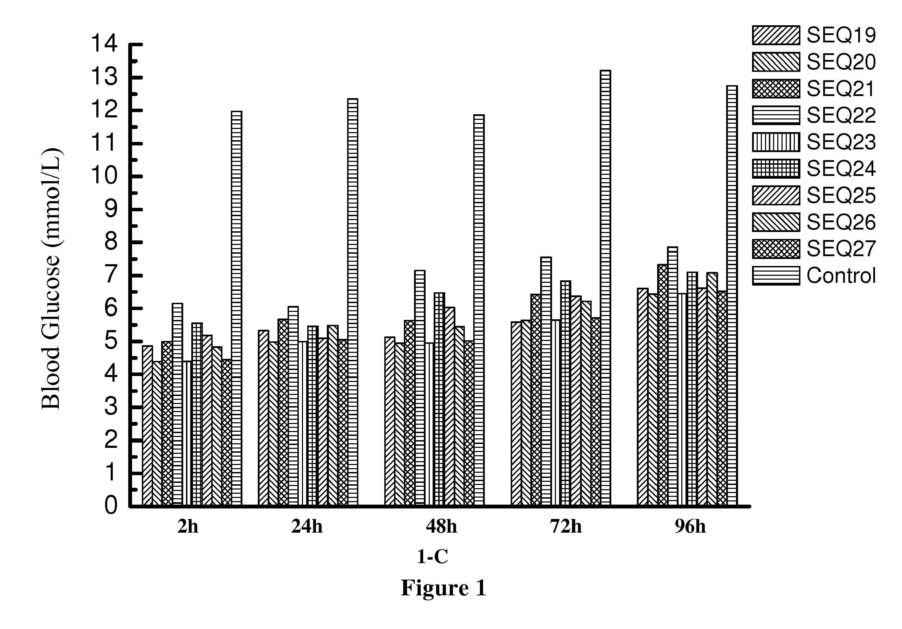

Figure 1 is the blood glucose reducing test of the GLP-1 analogue (SEQ ID NO 1-27) in Example 2; wherein Figure 1-A represents the blood glucose reducing test of the GLP-1 analogue (SEQ ID NO 1-9), in which ten data bars corresponding to each of the time points represent SEQ1-9 and the control successively from left to right; Figure 1-B represents the blood glucose reducing test of the GLP-1 analogue (SEQ ID NO 10-18), in which ten data bars corresponding to each of the time points represent SEQ10-18 and the control successively from left to right; Figure 1-C represents the blood glucose reducing test of the GLP-1 analogue (SEQ ID NO 19-27), in which ten data bars corresponding to each of the time points represent SEQ 19-27 and the control successively from left to right;

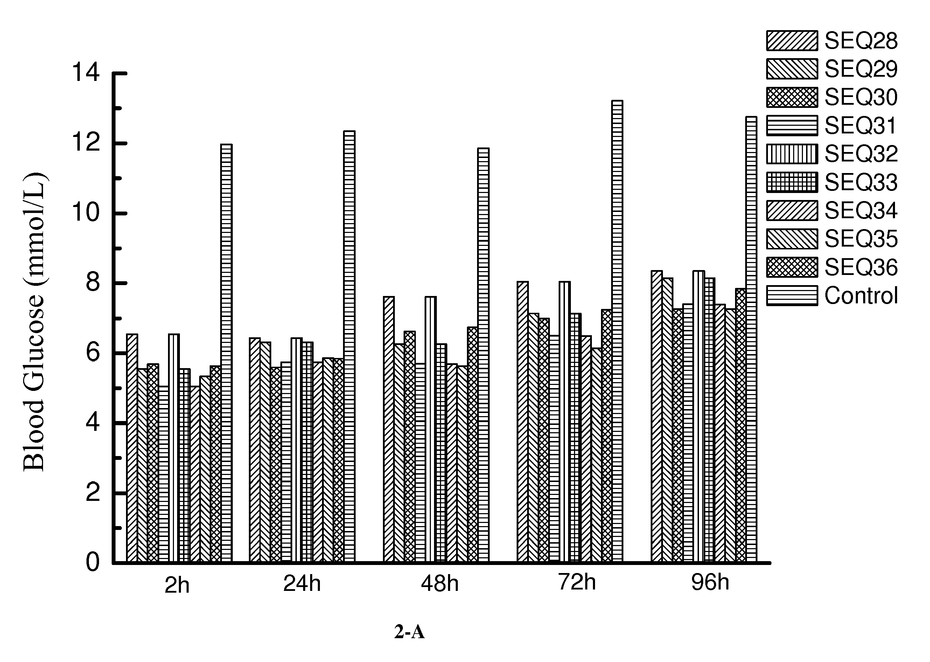

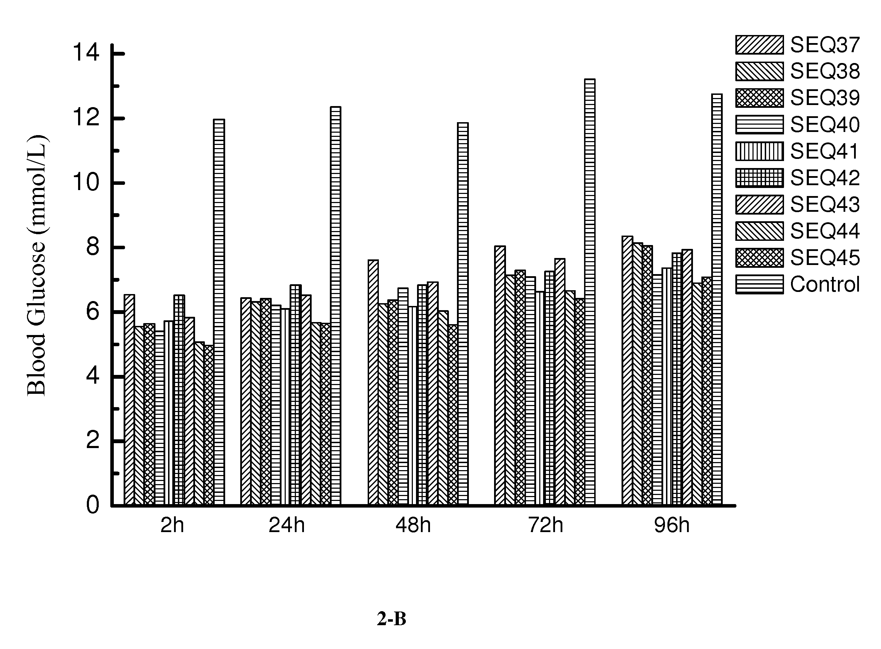

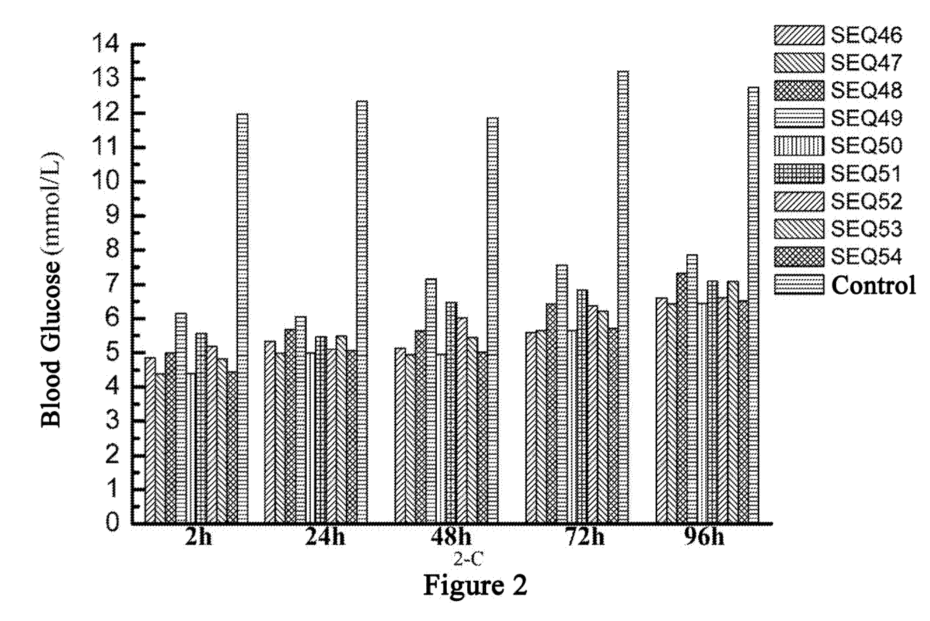

Figure 2 is the blood glucose reducing test of the GLP-1 analogue (SEQ ID NO 28-54) in Example 3; wherein Figure 2-A represents the blood glucose reducing test of the GLP-1 analogue (SEQ ID NO 28-36), in which ten data bars corresponding to each of the time points represent SEQ28-36 and the control successively from left to right; Figure 2-B represents the blood glucose reducing test of the GLP-1 analogue (SEQ ID NO 37-45), in which ten data bars corresponding to each of the time points represent SEQ37-45 and the control successively from left to right; Figure 2-C represents the blood glucose reducing test of the GLP-1 analogue (SEQ ID NO 46-54), in which ten data bars corresponding to each of the time points represent SEQ46-54 and the control successively from left to right;

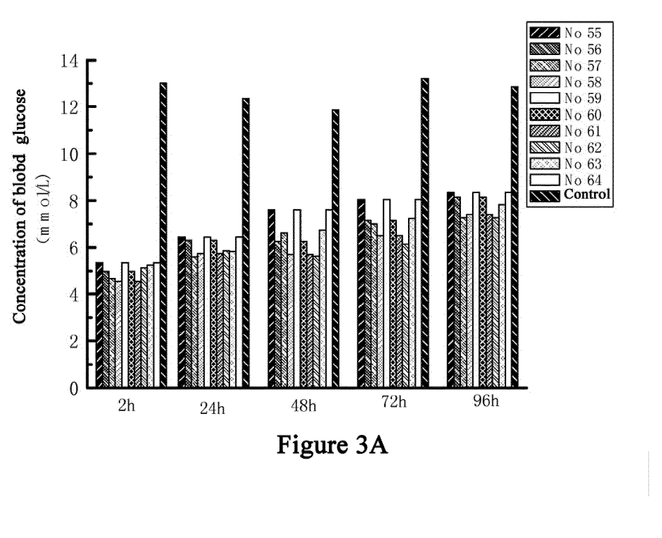

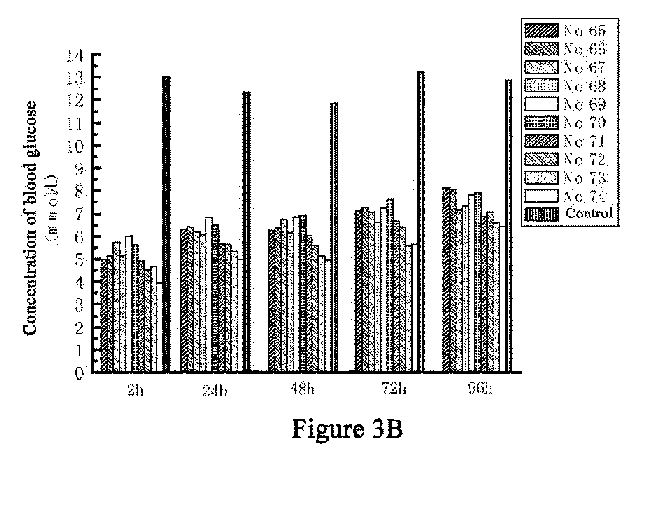

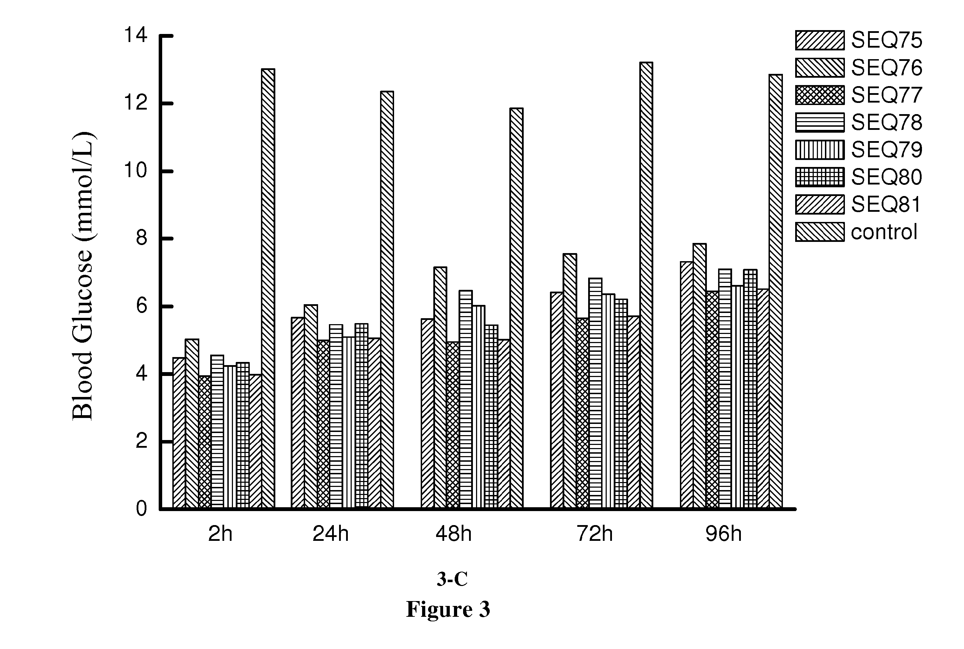

Figure 3 is the blood glucose reducing test of the GLP-1 analogue (SEQ ID NO 55-81) in Example 4; Figure 3-A represents the blood glucose reducing test of the GLP-1 analogue (SEQ ID NO 55-64), in which eleven bars corresponding to each of the time points represent SEQ55-64 and the control successively from left to right; Figure 3-B represents the blood glucose reducing test of the GLP-1 analogue (SEQ ID NO 65-74), in which eleven data bars corresponding to each of the time points represent SEQ65-74 and the control successively from left to right; Figure 3-C represents the blood glucose reducing test of the GLP-1 analogue (SEQ ID NO 75-81), in which eight data bars corresponding to each of the time points represent SEQ75-81 and the control successively from left to right;
Figure 4 represents the blood glucose reducing test of the GLP-1 analogue (SEQ ID NO 82-84) in Example 5, in which four bars corresponding to each of the time points represent SEQ82-84 and the control successively;
Figure 5 is a graph describing the stability of the GLP-1 analogue in the human serum in Example 6;
Figure 6 is a graph describing the stability of the GLP-1 analogue in the human serum in Example 7;
Figure 7 is a graph describing the stability of the GLP-1 analogue in the human serum in Example 8.

### SPECIFIC MODE FOR CARRYING OUT THE INVENTION

The present invention is now further described in detail in combination with the specific embodiments. The examples given are only for illustrating the present invention and not limiting the scope of the present invention.

In the following examples, various processes and methods which are not described in detail are conventional methods known in the art.

### Example 1: solid-phase synthesis of polypeptide

Using the method of solid phase polypeptide synthesis in accordance with Fmoc strategy, the synthesis of the polypeptides of the present invention is performed using the CS 336X type apparatus produced by CSBio Company. The method of synthesis is performed in accordance with the manufacturer's equipment instructions.

The obtained polypeptides are purified on a HPLC C18 semi-preparative column, using acetonitrile as the mobile phase. The freeze dried powder is obtained through desalination and lyophilization. All the polypeptides obtained in the present invention contain disulfide bonds, and ammonium bicarbonate or other reducing agent is used to form the disulfide bonds in the polypeptides.

### Example 2: related blood glucose reducing function of the GLP-1 analogue (general formula II)

The polypeptides used in this Example are as follows:

SEQ ID NO 1: ⁷HAEGT FTSCV SSYLE GQAAK EFIAW LVKGR G³⁷GGGGG
C GGG

SEQ ID NO 2: ^7HAEGT FTSCV SSYLE GQAAK EFIAW LVKGR G^37GGGGG
GGGGGC GGG

SEQ ID NO 3: ⁷HAEGT FTSCV SSYLE GQAAK EFIAW LVKGR G³⁷GGGGG
GGGGGGGGGC GGG

SEQ ID NO 4: ⁷HAEGT FTSCV SSYLE GQAAK EFIAW LVKGR G³⁷GGGGG
C GGGGG G

SEQ ID NO 5: ^7HAEGT FTSCV SSYLE GQAAK EFIAW LVKGR G^37GGGGG
C GGGGG GGGG

SEQ ID NO 6: ⁷HAEGT FTSCV SSYLE GQAAK EFIAW LVKGR G³⁷GGGGG
GGGGGC GGGGG G

SEQ ID NO 7: ⁷HAEGT FTSCV SSYLE GQAAK EFIAW LVKGR G³⁷GGGGG
GGGGGC GGGGG GGGG

SEQ ID NO 8: ⁷HAEGT FTSCV SSYLE GQAAK EFIAW LVKGR G³⁷GGGGG
GGGGGGGGGC GGGGG G

SEQ ID NO 9: ^7HAEGT FTSCV SSYLE GQAAK EFIAW LVKGR G^37GGGGG
GGGGGGGGGC GGGGG GGGG

SEQ ID NO 10: ⁷HAEGT FTSCV SSYLE GQAAK EFIAW LVKGR
G³⁷AAAAA C AAA

SEQ ID NO 11: ⁷HAEGT FTSCV SSYLE GQAAK EFIAW LVKGR
G³⁷AAAAA AAAAAC AAA

SEQ ID NO 12: ^7HAEGT FTSCV SSYLE GQAAK EFIAW LVKGR
G^37AAAAA AAAAAAAAAAC AAA

SEQ ID NO 13: ⁷HAEGT FTSCV SSYLE GQAAK EFIAW LVKGR
G³⁷AAAAA C AAAAA A

SEQ ID NO 14: ⁷HAEGT FTSCV SSYLE GQAAK EFIAW LVKGR
G³⁷AAAAA C AAAAA AAAA

SEQ ID NO 15: ^7HAEGT FTSCV SSYLE GQAAK EFIAW LVKGR
G^37AAAAA AAAAAC AAAAA A

SEQ ID NO 16: ⁷HAEGT FTSCV SSYLE GQAAK EFIAW LVKGR
G³⁷AAAAA AAAAAC AAAAA AAAA

SEQ ID NO 17: ⁷HAEGT FTSCV SSYLE GQAAK EFIAW LVKGR
G³⁷AAAAA AAAAAAAAAAC AAAAA A

SEQ ID NO 18: ⁷HAEGT FTSCV SSYLE GQAAK EFIAW LVKGR
G³⁷AAAAA AAAAAAAAAAC AAAAA AAAA

SEQ ID NO 19: ⁷HAEGT FTSCV SSYLE GQAAK EFIAW LVKGR
G³⁷VVVVV C VVV

SEQ ID NO 20: ⁷HAEGT FTSCV SSYLE GQAAK EFIAW LVKGR
G³⁷VVVVVV VVVVVC VVV

SEQ ID NO 21: ⁷HAEGT FTSCV SSYLE GQAAK EFIAW LVKGR
G³⁷VVVVV VVVVVVVVVVC VVV

SEQ ID NO 22: ⁷HAEGT FTSCV SSYLE GQAAK EFIAW LVKGR
G³⁷VVVVVV C VVVVVV V

SEQ ID NO 23: ⁷HAEGT FTSCV SSYLE GQAAK EFIAW LVKGR
G³⁷VVVVVV C VVVVVV VVVV

SEQ ID NO 24: ⁷HAEGT FTSCV SSYLE GQAAK EFIAW LVKGR
G³⁷VVVVVV VVVVVC VVVVVV V

SEQ ID NO 25: ⁷HAEGT FTSCV SSYLE GQAAK EFIAW LVKGR
G³⁷VVVVVV VVVVVC VVVVVV VVVV

SEQ ID NO 26: ⁷HAEGT FTSCV SSYLE GQAAK EFIAW LVKGR
G³⁷VVVVV VVVVVVVVVVC VVVVV V

SEQ ID NO 27: ⁷HAEGT FTSCV SSYLE GQAAK EFIAW LVKGR
G³⁷VVVVV VVVVVVVVVVC VVVVV VVVV

1 mg each of the above polypeptides is dissolved in 1 ml physiological saline to produce a polypeptide solution, and the polypeptide solution is subcutaneously injected into mice (200 µl per mouse, 6 mice per group, and the mice are purchased from Shanghai Laboratory Animal Center of Chinese Academy of Sciences). After 30 minutes form the subcutaneous injection, 400 microgram of glucose is injected into each mouse. The blood glucose levels of the mice are measured at 2 hours, 24 hours, 48 hours, 72 hours and 96 hours respectively after the injection of glucose (note: the same dosage of glucose is injected again at 2 hours before each measurement of the blood glucose). The results are shown in Figure 1, in which the control (blank) in this Example is the mice subcutaneous injected with 1ml physiological saline, and the injection of glucose is the same as the other test groups.

### Example 3: related blood glucose reducing function of the GLP-1 analogue (general formula III)

The polypeptides used in this Example are as follows:

SEQ ID NO 28: ⁷HAEGT FTSDV SSYLE GQAAK EFICW LVKGR G³⁷G C
GGGGG GGGGG

SEQ ID NO 29: ⁷HAEGT FTSDV SSYLE GQAAK EFICW LVKGR G³⁷G C
GGGGG GGGGGGGGG

SEQ ID NO 30: 7HAEGT FTSDV SSYLE GQAAK EFICW LVKGR G37G C
GGGGG GGGGGGGGGGGGGGGGGG

SEQ ID NO 31: ⁷HAEGT FTSDV SSYLE GQAAK EFICW LVKGR G³⁷GG C
GGGGG GGGGG

SEQ ID NO 32: ⁷HAEGT FTSDV SSYLE GQAAK EFICW LVKGR G³⁷GG C
GGGGG GGGGGGGGG

SEQ ID NO 33: ⁷HAEGT FTSDV SSYLE GQAAK EFICW LVKGR G³⁷GG C
GGGGG GGGGGGGGGGGGG

SEQ ID NO 34: ⁷HAEGT FTSDV SSYLE GQAAK EFICW LVKGR
G³⁷GGGGG C GGGGG GGGGG

SEQ ID NO 35: ⁷HAEGT FTSDV SSYLE GQAAK EFICW LVKGR
G³⁷GGGGG C GGGGG GGGGGGGGGG

SEQ ID NO 36: ⁷HAEGT FTSDV SSYLE GQAAK EFICW LVKGR
G³⁷GGGGG C GGGGG GGGGGGGGGGGGG

SEQ ID NO 37: ⁷HAEGT FTSDV SSYLE GQAAK EFICW LVKGR G³⁷A C
AAAAA AAAAA

SEQ ID NO 38: ⁷HAEGT FTSDV SSYLE GQAAK EFICW LVKGR G³⁷A C
AAAAA AAAAAAAAAA

SEQ ID NO 39: 7HAEGT FTSDV SSYLE GQAAK EFICW LVKGR G37A C
AAAAA AAAAAAAAAAAAAAAAAAAAAAA

SEQ ID NO 40: 7HAEGT FTSDV SSYLE GQAAK EFICW LVKGR G37AA C
AAAAA AAAAA

SEQ ID NO 41: ⁷HAEGT FTSDV SSYLE GQAAK EFICW LVKGR G³⁷AA C
AAAAA AAAAAAAAAA

SEQ ID NO 42: ⁷HAEGT FTSDV SSYLE GQAAK EFICW LVKGR G³⁷AA C
AAAAA AAAAAAAAAAAAAAAA

SEQ ID NO 43: ⁷HAEGT FTSDV SSYLE GQAAK EFICW LVKGR
G³⁷AAAAA C AAAAA AAAAA

SEQ ID NO 44: ⁷HAEGT FTSDV SSYLE GQAAK EFICW LVKGR
G³⁷AAAAA C AAAAA AAAAAAAAAA

SEQ ID NO 45: ⁷HAEGT FTSDV SSYLE GQAAK EFICW LVKGR
G³⁷AAAAA C AAAAA AAAAAAAAAAAAAAAA

SEQ ID NO 46: ⁷HAEGT FTSDV SSYLE GQAAK EFICW LVKGR G³⁷V C
VVVVVV VVVVVV

SEQ ID NO 47: ⁷HAEGT FTSDV SSYLE GQAAK EFICW LVKGR G³⁷V C
VVVVVV VVVVVVVVVVV

SEQ ID NO 48: ⁷HAEGT FTSDV SSYLE GQAAK EFICW LVKGR G³⁷V C
VVVVVV VVVVVVVVVVVVVVVVVVVVVV

SEQ ID NO 49: ⁷HAEGT FTSDV SSYLE GQAAK EFICW LVKGR G³⁷VV C
VVVVVV VVVVVV

SEQ ID NO 50: ⁷HAEGT FTSDV SSYLE GQAAK EFICW LVKGR G³⁷VV C
VVVVVV VVVVVVVVVVV

SEQ ID NO 51: ⁷HAEGT FTSDV SSYLE GQAAK EFICW LVKGR G³⁷VV C
VVVVVV VVVVVVVVVVVVVVVVV

SEQ ID NO 52: ⁷HAEGT FTSDV SSYLE GQAAK EFICW LVKGR
G³⁷VVVVVV C VVVVVV VVVVVV

SEQ ID NO 53: ⁷HAEGT FTSDV SSYLE GQAAK EFICW LVKGR
G³⁷VVVVVV C VVVVVV VVVVVVVVVVV

SEQ ID NO 54: ⁷HAEGT FTSDV SSYLE GQAAK EFICW LVKGR
G³⁷VVVVV C VVVVV VVVVVVVVVVVVVVVV

1 mg each of the above polypeptides is dissolved in 1 ml physiological saline to produce a polypeptide solution, and the polypeptide solution is subcutaneously injected into mice (200 µl per mouse, 6 mice per group, and the mice are purchased from Shanghai Laboratory Animal Center of Chinese Academy of Sciences). After 30 minutes from the subcutaneous injection, 400 microgram of glucose is injected into each mouse. The blood glucose levels of the mice are measured at 2 hours, 24 hours, 48 hours, 72 hours and 96 hours respectively after the injection of glucose (note: the same dosage of glucose is injected again at 2 hours before each measurement of the blood glucose). The results are shown in Figure 2, in which the control (blank) in this Example is the mice subcutaneous injected with 1ml physiological saline, and the injection of glucose is the same as the other test groups.

### Example 4: related blood glucose reducing function of the GLP-1 analogue (general formula IV)

The polypeptides used in this Example are as follows: 
SEQ ID NO 55: ⁷HAEGT FTSDV SSYLE GQAAK EFICW LCKGR G³⁷G C
GGGGG GGGGG
 
SEQ ID NO 56: ⁷HAEGT FTSDV SSYLE GQAAK EFICW LCKGR G³⁷G C
GGGGG GGGGGGGGG
 
SEQ ID NO 57: ⁷HAEGT FTSDV SSYLE GQAAK EFICW LCKGR G³⁷G C
GGGGG GGGGGGGGGGGGG
 
SEQ ID NO 58: ⁷HAEGT FTSDV SSYLE GQAAK EFICW LCKGR G³⁷GG C
GGGGG GGGGG
 
SEQ ID NO 59: ⁷HAEGT FTSDV SSYLE GQAAK EFICW LCKGR G³⁷GG C
GGGGG GGGGGGGGG
 
SEQ ID NO 60: ⁷HAEGT FTSDV SSYLE GQAAK EFICW LCKGR G³⁷GG C
GGGGG GGGGGGGGGGGGG
 
SEQ ID NO 61: ⁷HAEGT FTSDV SSYLE GQAAK EFICW LCKGR
G³⁷GGGGG C GGGGG GGGGG
 
SEQ ID NO 62: ⁷HAEGT FTSDV SSYLE GQAAK EFICW LCKGR
G³⁷GGGGG C GGGGG GGGGGGGGG
 
SEQ ID NO 63: ⁷HAEGT FTSDV SSYLE GQAAK EFICW LCKGR
G³⁷GGGGG C GGGGG GGGGGGGGGGGGG
 
SEQ ID NO 64: ⁷HAEGT FTSDV SSYLE GQAAK EFICW LCKGR G³⁷A C
AAAAA AAAAA
 
SEQ ID NO 65: ⁷HAEGT FTSDV SSYLE GQAAK EFICW LCKGR G³⁷A C
AAAAA AAAAAAAAAA
 
SEQ ID NO 66: ⁷HAEGT FTSDV SSYLE GQAAK EFICW LCKGR G³⁷A C
AAAAA AAAAAAAAAAAAAAA
 
SEQ ID NO 67: ⁷HAEGT FTSDV SSYLE GQAAK EFICW LCKGR G³⁷AA C
AAAAA AAAAA
 
SEQ ID NO 68: ⁷HAEGT FTSDV SSYLE GQAAK EFICW LCKGR G³⁷AA C
AAAAA AAAAAAAAAA
 
SEQ ID NO 69: ⁷HAEGT FTSDV SSYLE GQAAK EFICW LCKGR G³⁷AA C
AAAAA AAAAAAAAAAAAAAA
 
SEQ ID NO 70: 7HAEGT FTSDV SSYLE GQAAK EFICW LCKGR
G37AAAAA C AAAAA AAAAA
 
SEQ ID NO 71: ⁷HAEGT FTSDV SSYLE GQAAK EFICW LCKGR
G³⁷AAAAA C AAAAA AAAAAAAAAA
 
SEQ ID NO 72: ⁷HAEGT FTSDV SSYLE GQAAK EFICW LCKGR
G³⁷AAAAA C AAAAA AAAAAAAAAAAAAAA
 
SEQ ID NO 73: ⁷HAEGT FTSDV SSYLE GQAAK EFICW LCKGR G³⁷V C
VVVVVV VVVVVV
 
SEQ ID NO 74: ⁷HAEGT FTSDV SSYLE GQAAK EFICW LCKGR G³⁷V C
VVVVV VVVVVVVVVV
 
SEQ ID NO 75: ⁷HAEGT FTSDV SSYLE GQAAK EFICW LCKGR G³⁷V C
VVVVVV VVVVVVVVVVVVVVVVV
 
SEQ ID NO 76: ⁷HAEGT FTSDV SSYLE GQAAK EFICW LCKGR G³⁷VV C
VVVVVV VVVVVV
 
SEQ ID NO 77: ⁷HAEGT FTSDV SSYLE GQAAK EFICW LCKGR G³⁷VV C
VVVVVV VVVVVVVVVVV
 
SEQ ID NO 78: 7HAEGT FTSDV SSYLE GQAAK EFICW LCKGR G37VV C
VVVVVV VVVVVVVVVVVVVVVVV
 
SEQ ID NO 79: ⁷HAEGT FTSDV SSYLE GQAAK EFICW LCKGR
G³⁷VVVVVV C VVVVVV VVVVVV
 
SEQ ID NO 80: ⁷HAEGT FTSDV SSYLE GQAAK EFICW LCKGR
G³⁷VVVVVV C VVVVVV VVVVVVVVVVV
 
SEQ ID NO 81: ⁷HAEGT FTSDV SSYLE GQAAK EFICW LCKGR
G³⁷VVVVV C VVVVV VVVVVVVVVVVVVVVV
 1 mg each of the above polypeptides is dissolved in 1 ml of physiological saline to produce a polypeptide solution, and the polypeptide solution is subcutaneously injected into mice (200 µl per mouse, 6 mice per group, and the mice are purchased from Shanghai Laboratory Animal Center of Chinese Academy of Sciences). After 30 minutes from the subcutaneous injection, 400 microgram of glucose is injected into each mouse. The blood glucose levels of the mice are measured at 2 hours, 24 hours, 48 hours, 72 hours and 96 hours respectively after the injection of glucose (note: the same dosage of glucose is injected again at 2 hours before each measurement of the blood glucose). The results are shown in Figure 3, in which the control (blank) in this Example is the mice subcutaneous injected with 1ml physiological saline, and the injection of glucose is the same as the other test groups.

### Example 5: GLP-1 analogue based on mutation of amino acids at the other sites into cysteines

The polypeptides used in this Example are as follows:

SEQ ID NO 82: ⁷HAEGT FTSCV SSYLE GQAAK EFIAW LVKGR G³⁷
GGGGG GGGGG C GGGGG GGGGG

SEQ ID NO 83: ⁷HAEGT FTSDV SSYLE CQAAK EFIAW LVKGR G³⁷
GGGGG C GGGGG GGGGG

SEQ ID NO 84: ⁷HAEGT FTSDV SSYLE GQAAK EFICW LVKGR G³⁷ GGG
C GGGGG GGGGGGGGGGGGGG

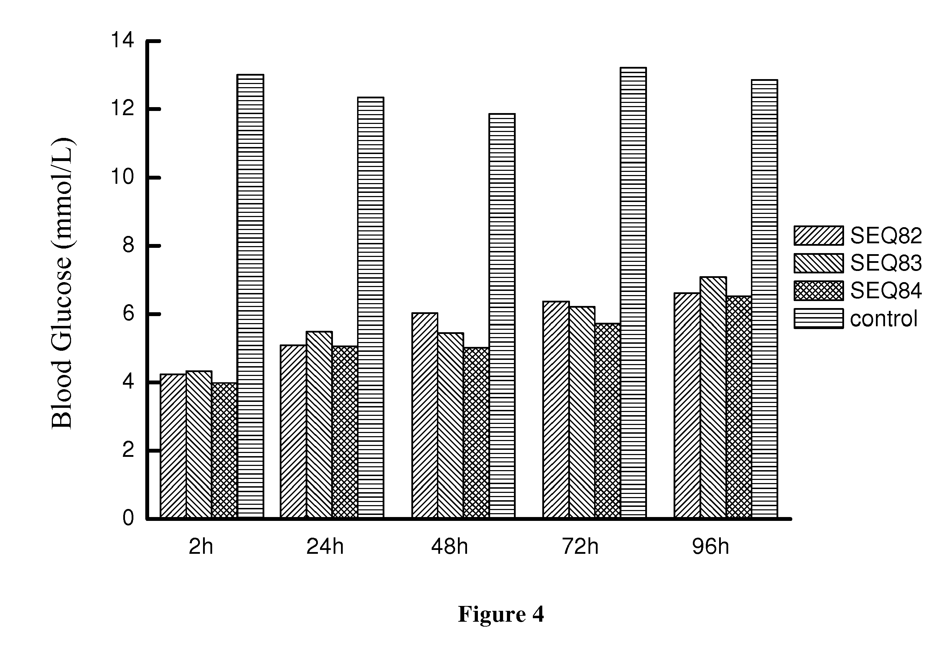

1 mg each of the above polypeptides is dissolved in 1 ml physiological saline, and subcutaneously injected into mice (200 µl per mouse, 6 mice per group, and the mice are purchased from Shanghai Laboratory Animal Center of Chinese Academy of Sciences). After 30 minutes from the subcutaneous injection, 400 mg glucose is injected into each mouse. The blood glucose levels of the mice are measured at 2 hours, 24 hours, 48 hours, 72 hours and 96 hours respectively after the injection of glucose. The results are shown in Figure 4, in which the control (blank) in this Example is the mice administered 1ml physiological saline.

### Example 6: Stability Determination of GLP-1 analogue in the human serum

The polypeptides used in this Example are as follows:

SEQ ID NO 58: ⁷HAEGT FTSDV SSYLE GQAAK EFICW LCKGR
G³⁷GG C GGGGG GGGGG

SEQ ID NO 59: ⁷HAEGT FTSDV SSYLE GQAAK EFICW LCKGR
G³⁷GG C GGGGG GGGGGGGGG

The blood samples are collected in triplicate from the volunteers using vacuum blood collection needles (BD Biosciences, Franlin Lakes, NJ), and then immediately centrifuged on a centrifuge at 13000 rpm for 20 minutes. The supernatant is taken for further use.

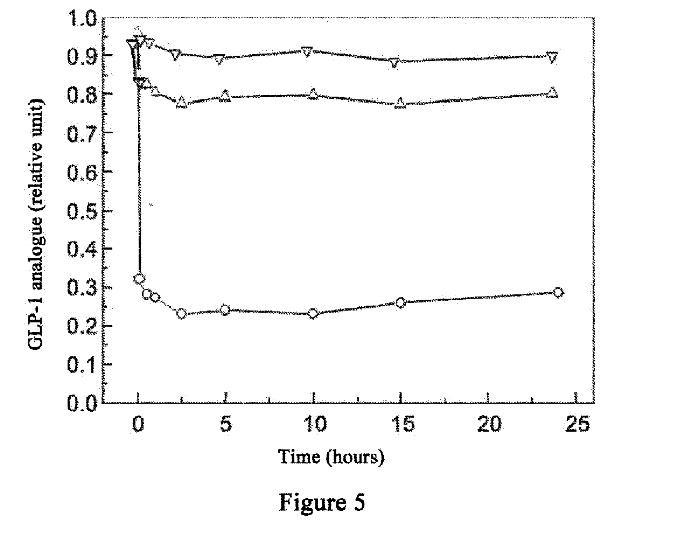

0.1 mg each of the above two polypeptides and the GLP-1 standard (which is purchased from Sangon Biotech of Shanghai, and has the sequences of HAEGTFTSDVSSYLEGQAAKEFIAWLVKGRG) are dissolved in 0.5 ml physiological saline, and added into 1 ml serum after thoroughly dissolution. The serum is labeled as blank group (0.1 mg GLP-1 is dissolved in 0.5 ml physiological saline), NO 58 test group (0.1 mg the peptide of the sequence of SEQ ID NO 58 is dissolved in 0.5 ml physiological saline), and NO 59 test group (0.1 mg the peptide of the sequence of SEQ ID NO 59 is dissolved in 0.5 ml physiological saline). After incubation at 37 °C for 0, 0.1, 0.3, 2.5, 5, 10, 15, and 24 h, 50 µl the serum mixtures of each group are detected with the GLP-1 enzyme-linked immune detection kits (which is purchased from ALPCO Immunoassays) respectively. The absorption value is read from the SpectraMax M5 microplate reader and used to calculate the concentrations of the GLP-1 analogue in different serum samples at different time points. The results are shown in Figure 5, in which the circle represents the blank group, the inverted triangle represents NO 58 test group, and regular triangle represents NO 59 test group. The results show that the half-lives of the No 58 and No 59 test group > 24 h.

### Example 7: Stability Determination of GLP-1 analogue in human serum

The polypeptides used in this Example are as follows:

SEQ ID NO 3: 7HAEGT FTSCV SSYLE GQAAK EFIAW LVKGR
G37GGGGG GGGGGGGGGC GGG

SEQ ID NO 25: ⁷HAEGT FTSCV SSYLE GQAAK EFIAW LVKGR
G³⁷VVVVV VVVVVC VVVVV VVVV

The blood samples are collected in triplicate from the volunteers using vacuum blood collection needles (BD Biosciences, Franlin Lakes, NJ), and then immediately centrifuged on a centrifuge at 13000 rpm for 20 minutes. The supernatant is taken for further use.

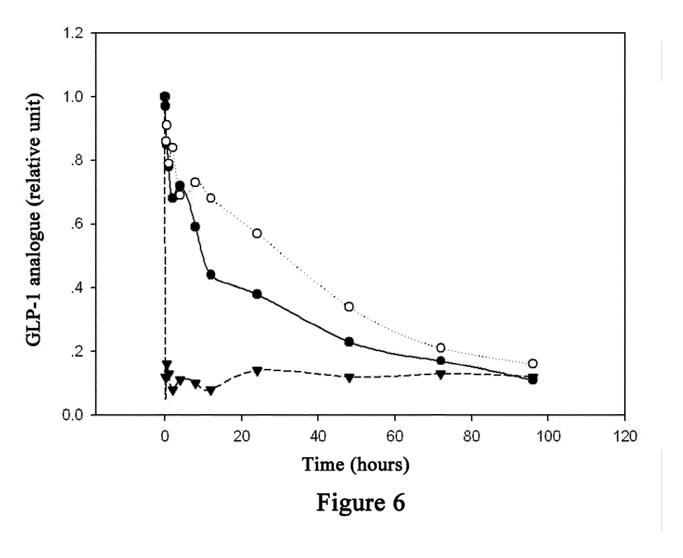

0.1 mg each of the above polypeptides and the GLP-1 standard (which is purchased from Sangon Biotech of Shanghai, and has the sequences of SEQ ID NO 85, HAEGTFTSDVSSYLEGQAAKEFIAWLVKGRG) are dissolved in 0.5 ml physiological saline, and added into serum after thoroughly dissolution. The serum is labeled as blank group (0.1 mg GLP-1 is dissolved in 0.5 ml physiological saline), NO. 3 test group (0.1 mg the peptide of the sequence of SEQ ID NO 3 is dissolved in 0.5 ml physiological saline), and NO 25 test group (0.1 mg the peptide of the sequence of SEQ ID NO 25 is dissolved in 0.5 ml physiological saline). After incubation at 37 °C for 0, 0.5, 1, 4, 8, 12, 24, 48, 72 and 96 h, 50 µl the serum mixtures of each group is detected with the GLP-1 enzyme-linked immune detection kits (which is purchased from ALPCO Immunoassays). The absorption value is read from the SpectraMax M5 microplate reader, and then used to calculate the concentrations of the GLP-1 analogue in different serum samples at different time points. The results are shown in Figure 6, in which the inverted triangle represents the blank group, the empty circle represents NO 3 test group, and the solid circle represents NO 25 test group. The results show that the half-lives of the No 3 and No 25 test groups are 48 h.

### Example 8: Stability Determination of GLP-1 analogue in human serum

The polypeptides used in this Example are as follows:

SEQ ID NO 28: ⁷HAEGT FTSDV SSYLE GQAAK EFICW LVKGR G³⁷G
C GGGGG GGGGG

SEQ ID NO 29: ⁷HAEGT FTSDV SSYLE GQAAK EFICW LVKGR G³⁷G
C GGGGG GGGGGGGGG

The blood samples are collected in triplicate from the volunteers using vacuum blood collection needles (BD Biosciences, Franlin Lakes, NJ), and then immediately centrifuged on a centrifuge at 13000 rpm for 20 minutes. The supernatant is taken for further use.

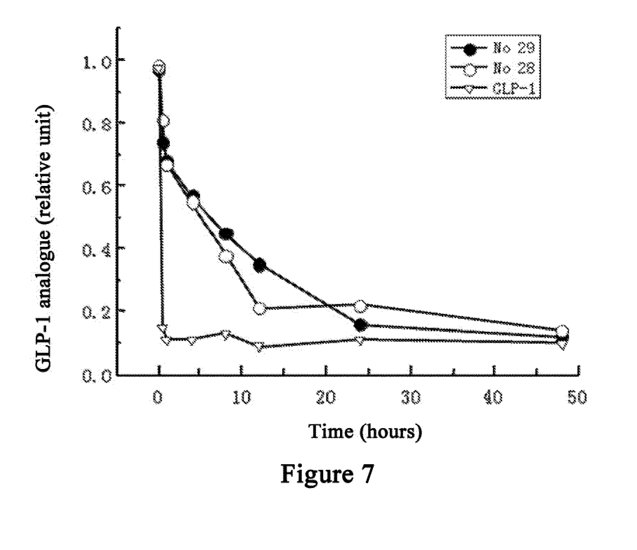

0.1 mg each of the above polypeptides and the GLP-1 standard (which is purchased from Sangon Biotech of Shanghai, and has the sequences of SEQ ID NO 85, HAEGTFTSDVSSYLEGQAAKEFIAWLVKGRG) are dissolved in 0.5 ml physiological saline, and added into the serum after thoroughly dissolution. The serum is labeled as blank group (0.1 mg GLP-1 is dissolved in 0.5 ml physiological saline), NO 28 test group (0.1 mg the peptide of the sequence of SEQ ID NO 28 is dissolved in 0.5 ml physiological saline), and NO 29 test group (0.1 mg the peptide of the sequence of SEQ ID NO 29 is dissolved in 0.5 ml physiological saline). After incubation at 37 °C for 0, 0.5, 1, 4, 8, 12, 24, and 48 h, 50µl the serum mixtures of each group is detected with the GLP-1 enzyme-linked immune detection kits (which is purchased from ALPCO Immunoassays) respectively. The absorption values are read from the SpectraMax M5 microplate reader, and used to calculate the concentrations of the GLP-1 analogue in different serum samples at different time points. The results are shown in Figure 7, in which the inverted triangle represents the blank group, the empty circle represents NO 28 test group, and the solid circle represents NO 29 test group. The results show that the half-lives of the No 28 and No 29 test groups are 24 h.

## Claims

1. A GLP-1 analogue having the following general formula A:
⁷HAEX₁₀T FTSX₁₅V SSYLE X₂₂QAAK EFIX₃₀W LX₃₃KGR G³⁷n₁X₁C n₂X₂ General Formula A,
wherein X₁₀ is glycine or cysteine, X₁₅ is aspartic acid or cysteine, X₂₂ is glycine or cysteine, X₃₀ is alanine or cysteine, X₃₃ is valine or cysteine, and at least one of X₁₀, X₁₅, X₂₂, X₃₀ and X₃₃ is cysteine;
n₁X₁ means that the number of X₁ is n₁, n₁=1-30, and X₁ is glycine, alanine or valine;
n₂X₂ means that the number of X₂ is n₂, n₂=0-30, and X₂ is glycine, alanine or valine; and
both of the two cysteines contained in the general formula A form disulfide bonds.

2. The GLP-1 analogue as claimed in claim 1, **characterized in that** the GLP-1 analogue has the following general formula I:
⁷HAEX₁₀T FTSX₁₅V SSYLE X₂₂QAAK EFIX₃₀W LX₃₃KGR G³⁷n₁X₁C n₂X₂ General Formula I,
wherein n₁=3-30, n₂=3-30;
preferably, the GLP-1 analogue is: 
SEQ ID NO 82: ⁷HAEGT FTSCV SSYLE GQAAK EFIAW LVKGR G³⁷
GGGGG GGGGG C GGGGG GGGGG;
 
SEQ ID NO 83: ⁷HAEGT FTSDV SSYLE CQAAK EFIAW LVKGR G³⁷
GGGGG C GGGGG GGGGG;
 or

SEQ ID NO 84: 7HAEGT FTSDV SSYLE GQAAK EFICW LVKGR G37
GGG C GGGGG GGGGGGGGGGGGGGG.

3. The GLP-1 analogue as claimed in claim 1, **characterized in that** the GLP-1 analogue has the following general formula II:
⁷HAEGT FTSCV SSYLE GQAAK EFIAW LVKGR G³⁷n₁X₁C n₂X₂ General Formula II,
n₁=1-20, n₂=0-25, and wherein X₁ is glycine, alanine or valine, X₂ is glycine, alanine or valine;
preferably, n₁=3-20, n₂=3-25;
further preferably, said n₁=5-15, n₂=3-9.

4. The GLP-1 analogue as claimed in claim 3, **characterized in that** the GLP-1 analogue is: 
SEQ ID NO 1: ⁷HAEGT FTSCV SSYLE GQAAK EFIAW LVKGR
G³⁷GGGGG C GGG;
 
SEQ ID NO 2: ⁷HAEGT FTSCV SSYLE GQAAK EFIAW LVKGR
G³⁷GGGGG GGGGGC GGG;
 
SEQ ID NO 3: 7HAEGT FTSCV SSYLE GQAAK EFIAW LVKGR
G37GGGGG GGGGGGGGGGC GGG;
 
SEQ ID NO 4: ⁷HAEGT FTSCV SSYLE GQAAK EFIAW LVKGR
G³⁷GGGGG C GGGGG G;
 
SEQ ID NO 5: ⁷HAEGT FTSCV SSYLE GQAAK EFIAW LVKGR
G³⁷GGGGG C GGGGG GGGG;
 
SEQ ID NO 6: ⁷HAEGT FTSCV SSYLE GQAAK EFIAW LVKGR
G³⁷GGGGG GGGGGC GGGGG G;
 
SEQ ID NO 7: ⁷HAEGT FTSCV SSYLE GQAAK EFIAW LVKGR
G³⁷GGGGG GGGGGC GGGGG GGGG;
 
SEQ ID NO 8: ⁷HAEGT FTSCV SSYLE GQAAK EFIAW LVKGR
G³⁷GGGGG GGGGGGGGGGC GGGGG G;
 
SEQ ID NO 9: ⁷HAEGT FTSCV SSYLE GQAAK EFIAW LVKGR
G³⁷GGGGG GGGGGGGGGC GGGGG GGGG;
 
SEQ ID NO 10: ⁷HAEGT FTSCV SSYLE GQAAK EFIAW LVKGR
G³⁷AAAAA C AAA;
 
SEQ ID NO 11: ⁷HAEGT FTSCV SSYLE GQAAK EFIAW LVKGR
G³⁷AAAAA AAAAAC AAA;
 
SEQ ID NO 12: ⁷HAEGT FTSCV SSYLE GQAAK EFIAW LVKGR
G³⁷AAAAA AAAAAAAAAAC AAA;
 
SEQ ID NO 13: ⁷HAEGT FTSCV SSYLE GQAAK EFIAW LVKGR
G³⁷AAAAA C AAAAA A;
 
SEQ ID NO 14: ⁷HAEGT FTSCV SSYLE GQAAK EFIAW LVKGR
G³⁷AAAAA C AAAAA AAAA;
 
SEQ ID NO 15: ⁷HAEGT FTSCV SSYLE GQAAK EFIAW LVKGR
G³⁷AAAAA AAAAAC AAAAA A;
 
SEQ ID NO 16: ⁷HAEGT FTSCV SSYLE GQAAK EFIAW LVKGR
G³⁷AAAAA AAAAAC AAAAA AAAA;
 
SEQ ID NO 17: ⁷HAEGT FTSCV SSYLE GQAAK EFIAW LVKGR
G³⁷AAAAA AAAAAAAAAC AAAAA A;
 
SEQ ID NO 18: ⁷HAEGT FTSCV SSYLE GQAAK EFIAW LVKGR
G³⁷AAAAA AAAAAAAAAC AAAAA AAAA;
 
SEQ ID NO 19: ⁷HAEGT FTSCV SSYLE GQAAK EFIAW LVKGR
G³⁷VVVVV C VVV;
 
SEQ ID NO 20: ⁷HAEGT FTSCV SSYLE GQAAK EFIAW LVKGR
G³⁷VVVVV VVVVVC VVV;
 
SEQ ID NO 21: ⁷HAEGT FTSCV SSYLE GQAAK EFIAW LVKGR
G³⁷VVVVV VVVVVVVVVVC VVV;
 
SEQ ID NO 22: ⁷HAEGT FTSCV SSYLE GQAAK EFIAW LVKGR
G³⁷VVVVVV C VVVVVV V;
 
SEQ ID NO 23: ⁷HAEGT FTSCV SSYLE GQAAK EFIAW LVKGR
G³⁷VVVVV C VVVVV VVVV;
 
SEQ ID NO 24: ^7HAEGT FTSCV SSYLE GQAAK EFIAW LVKGR
G^37VVVVV VVVVVC VVVVV V;
 
SEQ ID NO 25: ^7HAEGT FTSCV SSYLE GQAAK EFIAW LVKGR
G^37VVVVVV VVVVVC VVVVV VVVV;
 
SEQ ID NO 26: ⁷HAEGT FTSCV SSYLE GQAAK EFIAW LVKGR
G³⁷VVVVV VVVVVVVVVVC VVVVV V;
 or

SEQ ID NO 27: ⁷HAEGT FTSCV SSYLE GQAAK EFIAW LVKGR
G³⁷VVVVV VVVVVVVVVVC VVVVV VVVV.

5. The GLP-1 analogue as claimed in claim 1, **characterized in that** the GLP-1 analogue has the following general formula III:
⁷HAEGT FTSDV SSYLE GQAAK EFICW LVKGR G³⁷ n₁X₁ C n₂X₂ General Formula III,
wherein n₁=1-10, n₂=0-25, X₁ is glycine, alanine or valine, X₂ is glycine, alanine or valine; preferably, n₁=3-10, n₂=3-25;
or, n₁=1-5, n₂=10-20;
further preferably, n₁=3-5, n₂=10-20.

6. The GLP-1 analogue as claimed in claim 5, **characterized in that** the GLP-1 analogue is: 
SEQ ID NO 28: ⁷HAEGT FTSDV SSYLE GQAAK EFICW LVKGR G³⁷G
C GGGGG GGGGG;
 
SEQ ID NO 29: ⁷HAEGT FTSDV SSYLE GQAAK EFICW LVKGR G³⁷G
C GGGGG GGGGGGGGG;
 
SEQ ID NO 30: ⁷HAEGT FTSDV SSYLE GQAAK EFICW LVKGR G³⁷G
C GGGGG GGGGGGGGGGGGGGGGGGG;
 
SEQ ID NO 31: ⁷HAEGT FTSDV SSYLE GQAAK EFICW LVKGR
G³⁷GG C GGGGG GGGGG;
 
SEQ ID NO 32: ⁷HAEGT FTSDV SSYLE GQAAK EFICW LVKGR
G³⁷GG C GGGGG GGGGGGGGG;
 
SEQ ID NO 33: ⁷HAEGT FTSDV SSYLE GQAAK EFICW LVKGR
G³⁷GG C GGGGG GGGGGGGGGGGGGGG;
 
SEQ ID NO 34: ⁷HAEGT FTSDV SSYLE GQAAK EFICW LVKGR
G³⁷GGGGG C GGGGG GGGGG;
 
SEQ ID NO 35: ⁷HAEGT FTSDV SSYLE GQAAK EFICW LVKGR
G³⁷GGGGG C GGGGG GGGGGGGGGG;
 
SEQ ID NO 36: ⁷HAEGT FTSDV SSYLE GQAAK EFICW LVKGR
G³⁷GGGGG C GGGGG GGGGGGGGGGGGG;
 
SEQ ID NO 37: ⁷HAEGT FTSDV SSYLE GQAAK EFICW LVKGR G³⁷A
C AAAAA AAAAA;
 
SEQ ID NO 38: ⁷HAEGT FTSDV SSYLE GQAAK EFICW LVKGR G³⁷A
C AAAAA AAAAAAAAAA;
 
SEQ ID NO 39: ⁷HAEGT FTSDV SSYLE GQAAK EFICW LVKGR G³⁷A
C AAAAA AAAAAAAAAAAAAAAAAAAAAAAAAA;
 
SEQ ID NO 40: ⁷HAEGT FTSDV SSYLE GQAAK EFICW LVKGR
G³⁷AA C AAAAA AAAAA;
 
SEQ ID NO 41: ^7HAEGT FTSDV SSYLE GQAAK EFICW LVKGR
G^37AA C AAAAA AAAAAAAAAA;
 
SEQ ID NO 42: ⁷HAEGT FTSDV SSYLE GQAAK EFICW LVKGR
G³⁷AA C AAAAA AAAAAAAAAAAAAAAAA;
 
SEQ ID NO 43: ^7HAEGT FTSDV SSYLE GQAAK EFICW LVKGR
G^37AAAAA C AAAAA AAAAA;
 
SEQ ID NO 44: ⁷HAEGT FTSDV SSYLE GQAAK EFICW LVKGR
G³⁷AAAAA C AAAAA AAAAAAAAAA;
 
SEQ ID NO 45: ⁷HAEGT FTSDV SSYLE GQAAK EFICW LVKGR
G³⁷AAAAA C AAAAA AAAAAAAAAAAAAAAAA;
 
SEQ ID NO 46: ^7HAEGT FTSDV SSYLE GQAAK EFICW LVKGR G^37V
C VVVVVV VVVVVV;
 
SEQ ID NO 47: 7HAEGT FTSDV SSYLE GQAAK EFICW LVKGR G37V
C VVVVVV VVVVVVVVVVV;
 
SEQ ID NO 48: ⁷HAEGT FTSDV SSYLE GQAAK EFICW LVKGR G³⁷V
C VVVVVV VVVVVVVVVVVVVVVVVVVVVVVVV;
 
SEQ ID NO 49: ⁷HAEGT FTSDV SSYLE GQAAK EFICW LVKGR
G³⁷VV C VVVVV VVVVV;
 
SEQ ID NO 50: ⁷HAEGT FTSDV SSYLE GQAAK EFICW LVKGR
G³⁷VV C VVVVV VVVVVVVVVV;
 
SEQ ID NO 51: ⁷HAEGT FTSDV SSYLE GQAAK EFICW LVKGR
G³⁷VV C VVVVVV VVVVVVVVVVVVVVVVVVV;
 
SEQ ID NO 52: ⁷HAEGT FTSDV SSYLE GQAAK EFICW LVKGR
G³⁷VVVVVV C VVVVVV VVVVVV;
 
SEQ ID NO 53: ⁷HAEGT FTSDV SSYLE GQAAK EFICW LVKGR
G³⁷VVVVVV C VVVVVV VVVVVVVVVVV;
 or

SEQ ID NO 54: 7HAEGT FTSDV SSYLE GQAAK EFICW LVKGR
G37VVVVVV C VVVVVV VVVVVVVVVVVVVVVVV.

7. The GLP-1 analogue as claimed in claim 1, **characterized in that** the GLP-1 analogue has the following general formula IV:
⁷HAEGT FTSDV SSYLE GQAAK EFICW LCKGR G³⁷ n₁X₁ C n₂X₂ General Formula IV,
wherein n₁=1-10, n₂=0-30, X₁ is glycine, alanine or valine, X₂ is glycine, alanine or valine; preferably, n₁=3-10, n₂=3-30;
or, n₁=1-5, n₂=10-20;
further preferably, n₁=3-5, n₂=10-20.

8. The GLP-1 analogue as claimed in claim 7, **characterized in that** the GLP-1 analogue is: SEQ ID NO 55: 7HAEGT FTSDV SSYLE GQAAK EFICW LCKGR G37G C GGGGG GGGGG; 
SEQ ID NO 56: ⁷HAEGT FTSDV SSYLE GQAAK EFICW LCKGR G³⁷G
C GGGGG GGGGGGGGG;
 
SEQ ID NO 57: ⁷HAEGT FTSDV SSYLE GQAAK EFICW LCKGR G³⁷G
C GGGGG GGGGGGGGGGGGG;
 
SEQ ID NO 58: ⁷HAEGT FTSDV SSYLE GQAAK EFICW LCKGR
G³⁷GG C GGGGG GGGGG;
 
SEQ ID NO 59: ^7HAEGT FTSDV SSYLE GQAAK EFICW LCKGR
G^37GG C GGGGG GGGGGGGGG;
 
SEQ ID NO 60: ⁷HAEGT FTSDV SSYLE GQAAK EFICW LCKGR
G³⁷GG C GGGGG GGGGGGGGGGGGGG;
 
SEQ ID NO 61: ⁷HAEGT FTSDV SSYLE GQAAK EFICW LCKGR
G³⁷GGGGG C GGGGG GGGGG;
 
SEQ ID NO 62: ⁷HAEGT FTSDV SSYLE GQAAK EFICW LCKGR
G³⁷GGGGG C GGGGG GGGGGGGGG;
 
SEQ ID NO 63: ⁷HAEGT FTSDV SSYLE GQAAK EFICW LCKGR
G³⁷GGGGG C GGGGG GGGGGGGGGGGGG;
 
SEQ ID NO 64: ⁷HAEGT FTSDV SSYLE GQAAK EFICW LCKGR G³⁷A
C AAAAA AAAAA;
 
SEQ ID NO 65: ⁷HAEGT FTSDV SSYLE GQAAK EFICW LCKGR G³⁷A
C AAAAA AAAAAAAAAA;
 
SEQ ID NO 66: ⁷HAEGT FTSDV SSYLE GQAAK EFICW LCKGR G³⁷A
C AAAAA AAAAAAAAAAAAAAA;
 
SEQ ID NO 67: ⁷HAEGT FTSDV SSYLE GQAAK EFICW LCKGR
G³⁷AA C AAAAA AAAAA;
 
SEQ ID NO 68: ⁷HAEGT FTSDV SSYLE GQAAK EFICW LCKGR
G³⁷AA C AAAAA AAAAAAAAAA;
 
SEQ ID NO 69: ⁷HAEGT FTSDV SSYLE GQAAK EFICW LCKGR
G³⁷AA C AAAAA AAAAAAAAAAAAAAAAA;
 
SEQ ID NO 70: ⁷HAEGT FTSDV SSYLE GQAAK EFICW LCKGR
G³⁷AAAAA C AAAAA AAAAA;
 
SEQ ID NO 71: ⁷HAEGT FTSDV SSYLE GQAAK EFICW LCKGR
G³⁷AAAAA C AAAAA AAAAAAAAAA;
 
SEQ ID NO 72: 7HAEGT FTSDV SSYLE GQAAK EFICW LCKGR
G37AAAAA C AAAAA AAAAAAAAAAAAAAAAA;
 
SEQ ID NO 73: ⁷HAEGT FTSDV SSYLE GQAAK EFICW LCKGR G³⁷V
C VVVVV VVVVV;
 
SEQ ID NO 74: ⁷HAEGT FTSDV SSYLE GQAAK EFICW LCKGR G³⁷V
C VVVVVV VVVVVVVVVV;
 
SEQ ID NO 75: ⁷HAEGT FTSDV SSYLE GQAAK EFICW LCKGR G³⁷V
C VVVVVV VVVVVVVVVVVVVVVVVV;
 
SEQ ID NO 76: ⁷HAEGT FTSDV SSYLE GQAAK EFICW LCKGR
G³⁷VV C VVVVVV VVVVVV;
 
SEQ ID NO 77: ⁷HAEGT FTSDV SSYLE GQAAK EFICW LCKGR
G³⁷VV C VVVVV VVVVVVVVVV;
 
SEQ ID NO 78: ⁷HAEGT FTSDV SSYLE GQAAK EFICW LCKGR
G³⁷VV C VVVVVV VVVVVVVVVVVVVVVVV;
 
SEQ ID NO 79: ⁷HAEGT FTSDV SSYLE GQAAK EFICW LCKGR
G³⁷VVVVVV C VVVVVV VVVVVV;
 
SEQ ID NO 80: ⁷HAEGT FTSDV SSYLE GQAAK EFICW LCKGR
G³⁷VVVVVV C VVVVVV VVVVVVVVVVV;
 or

SEQ ID NO 81: ⁷HAEGT FTSDV SSYLE GQAAK EFICW LCKGR
G³⁷VVVVVV C VVVVVV VVVVVVVVVVVVVVVVV.

9. A preparation method of the GLP-1 analogue as claimed in any one of claims 1-8 comprising solid phase polypeptide synthesis in accordance with Fmoc strategy.

10. Use of the GLP-1 analogue as claimed in any one of claims 1-8 in the manufacture of a medicament for treating diabetes, obesity and/or diseases associated with diabetes and diseases associated with obesity.

11. Use of the GLP-1 analogue as claimed in any one of claims 1-8 in the manufacture of a medicament for preventing obesity.

12. A pharmaceutical composition comprising the GLP-1 analogue as claimed in any one of claims 1-8 and one or more pharmaceutically acceptable auxiliary materials;
Preferably, the pharmaceutical composition is an injection, further preferably is a lyophilized powder or a solution for injection.


SEQUENCE LISTING

<110>  Tianjin Institue of Pharmaceutical Research

<120>  A GLP-1 ANALOGUE, PREPARATION METHODS AND USE THEREOF

<130>  EIC11310020P-EP

<160>  85

<170>  PatentIn version 3.3

<210>  1
<211>  40
<212>  PRT
<213>  Artificial Sequence

<400>  1

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Gly
            20                  25                  30

Gly Gly Gly Gly Cys Gly Gly Gly
        35                  40

<210>  2
<211>  45
<212>  PRT
<213>  Artificial Sequence

<400>  2

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Gly
            20                  25                  30

Gly Gly Gly Gly Gly Gly Gly Gly Gly Cys Gly Gly Gly
        35                  40                  45

<210>  3
<211>  50
<212>  PRT
<213>  Artificial Sequence

<400>  3

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Gly
            20                  25                  30

Gly Gly Gly Gly Gly Gly Gly Gly Gly Gly Gly Gly Gly Gly Cys Gly
        35                  40                  45

Gly Gly
    50

<210>  4
<211>  43
<212>  PRT
<213>  Artificial Sequence

<400>  4

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Gly
            20                  25                  30

Gly Gly Gly Gly Cys Gly Gly Gly Gly Gly Gly
        35                  40

<210>  5
<211>  46
<212>  PRT
<213>  Artificial Sequence

<400>  5

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Gly
            20                  25                  30

Gly Gly Gly Gly Cys Gly Gly Gly Gly Gly Gly Gly Gly Gly
        35                  40                  45

<210>  6
<211>  48
<212>  PRT
<213>  Artificial Sequence

<400>  6

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Gly
            20                  25                  30

Gly Gly Gly Gly Gly Gly Gly Gly Gly Cys Gly Gly Gly Gly Gly Gly
        35                  40                  45

<210>  7
<211>  51
<212>  PRT
<213>  Artificial Sequence

<400>  7

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Gly
            20                  25                  30

Gly Gly Gly Gly Gly Gly Gly Gly Gly Cys Gly Gly Gly Gly Gly Gly
        35                  40                  45

Gly Gly Gly
    50

<210>  8
<211>  53
<212>  PRT
<213>  Artificial Sequence

<400>  8

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Gly
            20                  25                  30

Gly Gly Gly Gly Gly Gly Gly Gly Gly Gly Gly Gly Gly Gly Cys Gly
        35                  40                  45

Gly Gly Gly Gly Gly
    50

<210>  9
<211>  56
<212>  PRT
<213>  Artificial Sequence

<400>  9

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Gly
            20                  25                  30

Gly Gly Gly Gly Gly Gly Gly Gly Gly Gly Gly Gly Gly Gly Cys Gly
        35                  40                  45

Gly Gly Gly Gly Gly Gly Gly Gly
    50                  55

<210>  10
<211>  40
<212>  PRT
<213>  Artificial Sequence

<400>  10

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Ala Ala Ala Cys Ala Ala Ala
        35                  40

<210>  11
<211>  45
<212>  PRT
<213>  Artificial Sequence

<400>  11

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Ala Ala Ala Ala Ala Ala Ala Ala Cys Ala Ala Ala
        35                  40                  45

<210>  12
<211>  50
<212>  PRT
<213>  Artificial Sequence

<400>  12

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Ala Ala Ala Ala Ala Ala Ala Ala Ala Ala Ala Ala Ala Cys Ala
        35                  40                  45

Ala Ala
    50

<210>  13
<211>  43
<212>  PRT
<213>  Artificial Sequence

<400>  13

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Ala Ala Ala Cys Ala Ala Ala Ala Ala Ala
        35                  40

<210>  14
<211>  46
<212>  PRT
<213>  Artificial Sequence

<400>  14

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Ala Ala Ala Cys Ala Ala Ala Ala Ala Ala Ala Ala Ala
        35                  40                  45

<210>  15
<211>  48
<212>  PRT
<213>  Artificial Sequence

<400>  15

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Ala Ala Ala Ala Ala Ala Ala Ala Cys Ala Ala Ala Ala Ala Ala
        35                  40                  45

<210>  16
<211>  51
<212>  PRT
<213>  Artificial Sequence

<400>  16

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Ala Ala Ala Ala Ala Ala Ala Ala Cys Ala Ala Ala Ala Ala Ala
        35                  40                  45

Ala Ala Ala
    50

<210>  17
<211>  53
<212>  PRT
<213>  Artificial Sequence

<400>  17

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Ala Ala Ala Ala Ala Ala Ala Ala Ala Ala Ala Ala Ala Cys Ala
        35                  40                  45

Ala Ala Ala Ala Ala
    50

<210>  18
<211>  56
<212>  PRT
<213>  Artificial Sequence

<400>  18

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Ala
            20                  25                  30

Ala Ala Ala Ala Ala Ala Ala Ala Ala Ala Ala Ala Ala Ala Cys Ala
        35                  40                  45

Ala Ala Ala Ala Ala Ala Ala Ala
    50                  55

<210>  19
<211>  40
<212>  PRT
<213>  Artificial Sequence

<400>  19

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Val
            20                  25                  30

Val Val Val Val Cys Val Val Val
        35                  40

<210>  20
<211>  45
<212>  PRT
<213>  Artificial Sequence

<400>  20

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Val
            20                  25                  30

Val Val Val Val Val Val Val Val Val Cys Val Val Val
        35                  40                  45

<210>  21
<211>  50
<212>  PRT
<213>  Artificial Sequence

<400>  21

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Val
            20                  25                  30

Val Val Val Val Val Val Val Val Val Val Val Val Val Val Cys Val
        35                  40                  45

Val Val
    50

<210>  22
<211>  43
<212>  PRT
<213>  Artificial Sequence

<400>  22

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Val
            20                  25                  30

Val Val Val Val Cys Val Val Val Val Val Val
        35                  40

<210>  23
<211>  46
<212>  PRT
<213>  Artificial Sequence

<400>  23

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Val
            20                  25                  30

Val Val Val Val Cys Val Val Val Val Val Val Val Val Val
        35                  40                  45

<210>  24
<211>  48
<212>  PRT
<213>  Artificial Sequence

<400>  24

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Val
            20                  25                  30

Val Val Val Val Val Val Val Val Val Cys Val Val Val Val Val Val
        35                  40                  45

<210>  25
<211>  51
<212>  PRT
<213>  Artificial Sequence

<400>  25

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Val
            20                  25                  30

Val Val Val Val Val Val Val Val Val Cys Val Val Val Val Val Val
        35                  40                  45

Val Val Val
    50

<210>  26
<211>  53
<212>  PRT
<213>  Artificial Sequence

<400>  26

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Val
            20                  25                  30

Val Val Val Val Val Val Val Val Val Val Val Val Val Val Cys Val
        35                  40                  45

Val Val Val Val Val
    50

<210>  27
<211>  56
<212>  PRT
<213>  Artificial Sequence

<400>  27

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Val
            20                  25                  30

Val Val Val Val Val Val Val Val Val Val Val Val Val Val Cys Val
        35                  40                  45

Val Val Val Val Val Val Val Val
    50                  55

<210>  28
<211>  43
<212>  PRT
<213>  Artificial Sequence

<400>  28

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Gly
            20                  25                  30

Cys Gly Gly Gly Gly Gly Gly Gly Gly Gly Gly
        35                  40

<210>  29
<211>  48
<212>  PRT
<213>  Artificial Sequence

<400>  29

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Gly
            20                  25                  30

Cys Gly Gly Gly Gly Gly Gly Gly Gly Gly Gly Gly Gly Gly Gly Gly
        35                  40                  45

<210>  30
<211>  58
<212>  PRT
<213>  Artificial Sequence

<400>  30

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Gly
            20                  25                  30

Cys Gly Gly Gly Gly Gly Gly Gly Gly Gly Gly Gly Gly Gly Gly Gly
        35                  40                  45

Gly Gly Gly Gly Gly Gly Gly Gly Gly Gly
    50                  55

<210>  31
<211>  44
<212>  PRT
<213>  Artificial Sequence

<400>  31

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Gly
            20                  25                  30

Gly Cys Gly Gly Gly Gly Gly Gly Gly Gly Gly Gly
        35                  40

<210>  32
<211>  49
<212>  PRT
<213>  Artificial Sequence

<400>  32

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Gly
            20                  25                  30

Gly Cys Gly Gly Gly Gly Gly Gly Gly Gly Gly Gly Gly Gly Gly Gly
        35                  40                  45

Gly

<210>  33
<211>  54
<212>  PRT
<213>  Artificial Sequence

<400>  33

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Gly
            20                  25                  30

Gly Cys Gly Gly Gly Gly Gly Gly Gly Gly Gly Gly Gly Gly Gly Gly
        35                  40                  45

Gly Gly Gly Gly Gly Gly
    50

<210>  34
<211>  47
<212>  PRT
<213>  Artificial Sequence

<400>  34

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Gly
            20                  25                  30

Gly Gly Gly Gly Cys Gly Gly Gly Gly Gly Gly Gly Gly Gly Gly
        35                  40                  45

<210>  35
<211>  52
<212>  PRT
<213>  Artificial Sequence

<400>  35

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Gly
            20                  25                  30

Gly Gly Gly Gly Cys Gly Gly Gly Gly Gly Gly Gly Gly Gly Gly Gly
        35                  40                  45

Gly Gly Gly Gly
    50

<210>  36
<211>  57
<212>  PRT
<213>  Artificial Sequence

<400>  36

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Gly
            20                  25                  30

Gly Gly Gly Gly Cys Gly Gly Gly Gly Gly Gly Gly Gly Gly Gly Gly
        35                  40                  45

Gly Gly Gly Gly Gly Gly Gly Gly Gly
    50                  55

<210>  37
<211>  43
<212>  PRT
<213>  Artificial Sequence

<400>  37

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Ala
            20                  25                  30

Cys Ala Ala Ala Ala Ala Ala Ala Ala Ala Ala
        35                  40

<210>  38
<211>  48
<212>  PRT
<213>  Artificial Sequence

<400>  38

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Ala
            20                  25                  30

Cys Ala Ala Ala Ala Ala Ala Ala Ala Ala Ala Ala Ala Ala Ala Ala
        35                  40                  45

<210>  39
<211>  58
<212>  PRT
<213>  Artificial Sequence

<400>  39

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Ala
            20                  25                  30

Cys Ala Ala Ala Ala Ala Ala Ala Ala Ala Ala Ala Ala Ala Ala Ala
        35                  40                  45

Ala Ala Ala Ala Ala Ala Ala Ala Ala Ala
    50                  55

<210>  40
<211>  44
<212>  PRT
<213>  Artificial Sequence

<400>  40

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Ala
            20                  25                  30

Ala Cys Ala Ala Ala Ala Ala Ala Ala Ala Ala Ala
        35                  40

<210>  41
<211>  49
<212>  PRT
<213>  Artificial Sequence

<400>  41

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Ala
            20                  25                  30

Ala Cys Ala Ala Ala Ala Ala Ala Ala Ala Ala Ala Ala Ala Ala Ala
        35                  40                  45

Ala

<210>  42
<211>  54
<212>  PRT
<213>  Artificial Sequence

<400>  42

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Ala
            20                  25                  30

Ala Cys Ala Ala Ala Ala Ala Ala Ala Ala Ala Ala Ala Ala Ala Ala
        35                  40                  45

Ala Ala Ala Ala Ala Ala
    50

<210>  43
<211>  47
<212>  PRT
<213>  Artificial Sequence

<400>  43

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Ala
            20                  25                  30

Ala Ala Ala Ala Cys Ala Ala Ala Ala Ala Ala Ala Ala Ala Ala
        35                  40                  45

<210>  44
<211>  52
<212>  PRT
<213>  Artificial Sequence

<400>  44

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Ala
            20                  25                  30

Ala Ala Ala Ala Cys Ala Ala Ala Ala Ala Ala Ala Ala Ala Ala Ala
        35                  40                  45

Ala Ala Ala Ala
    50

<210>  45
<211>  57
<212>  PRT
<213>  Artificial Sequence

<400>  45

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Ala
            20                  25                  30

Ala Ala Ala Ala Cys Ala Ala Ala Ala Ala Ala Ala Ala Ala Ala Ala
        35                  40                  45

Ala Ala Ala Ala Ala Ala Ala Ala Ala
    50                  55

<210>  46
<211>  43
<212>  PRT
<213>  Artificial Sequence

<400>  46

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Val
            20                  25                  30

Cys Val Val Val Val Val Val Val Val Val Val
        35                  40

<210>  47
<211>  48
<212>  PRT
<213>  Artificial Sequence

<400>  47

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Val
            20                  25                  30

Cys Val Val Val Val Val Val Val Val Val Val Val Val Val Val Val
        35                  40                  45

<210>  48
<211>  58
<212>  PRT
<213>  Artificial Sequence

<400>  48

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Val
            20                  25                  30

Cys Val Val Val Val Val Val Val Val Val Val Val Val Val Val Val
        35                  40                  45

Val Val Val Val Val Val Val Val Val Val
    50                  55

<210>  49
<211>  44
<212>  PRT
<213>  Artificial Sequence

<400>  49

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Val
            20                  25                  30

Val Cys Val Val Val Val Val Val Val Val Val Val
        35                  40

<210>  50
<211>  49
<212>  PRT
<213>  Artificial Sequence

<400>  50

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Val
            20                  25                  30

Val Cys Val Val Val Val Val Val Val Val Val Val Val Val Val Val
        35                  40                  45

Val

<210>  51
<211>  54
<212>  PRT
<213>  Artificial Sequence

<400>  51

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Val
            20                  25                  30

Val Cys Val Val Val Val Val Val Val Val Val Val Val Val Val Val
        35                  40                  45

Val Val Val Val Val Val
    50

<210>  52
<211>  47
<212>  PRT
<213>  Artificial Sequence

<400>  52

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Val
            20                  25                  30

Val Val Val Val Cys Val Val Val Val Val Val Val Val Val Val
        35                  40                  45

<210>  53
<211>  52
<212>  PRT
<213>  Artificial Sequence

<400>  53

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Val
            20                  25                  30

Val Val Val Val Cys Val Val Val Val Val Val Val Val Val Val Val
        35                  40                  45

Val Val Val Val
    50

<210>  54
<211>  57
<212>  PRT
<213>  Artificial Sequence

<400>  54

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Val
            20                  25                  30

Val Val Val Val Cys Val Val Val Val Val Val Val Val Val Val Val
        35                  40                  45

Val Val Val Val Val Val Val Val Val
    50                  55

<210>  55
<211>  43
<212>  PRT
<213>  Artificial Sequence

<400>  55

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Gly
            20                  25                  30

Cys Gly Gly Gly Gly Gly Gly Gly Gly Gly Gly
        35                  40

<210>  56
<211>  48
<212>  PRT
<213>  Artificial Sequence

<400>  56

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Gly
            20                  25                  30

Cys Gly Gly Gly Gly Gly Gly Gly Gly Gly Gly Gly Gly Gly Gly Gly
        35                  40                  45

<210>  57
<211>  53
<212>  PRT
<213>  Artificial Sequence

<400>  57

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Gly
            20                  25                  30

Cys Gly Gly Gly Gly Gly Gly Gly Gly Gly Gly Gly Gly Gly Gly Gly
        35                  40                  45

Gly Gly Gly Gly Gly
    50

<210>  58
<211>  44
<212>  PRT
<213>  Artificial Sequence

<400>  58

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Gly
            20                  25                  30

Gly Cys Gly Gly Gly Gly Gly Gly Gly Gly Gly Gly
        35                  40

<210>  59
<211>  49
<212>  PRT
<213>  Artificial Sequence

<400>  59

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Gly
            20                  25                  30

Gly Cys Gly Gly Gly Gly Gly Gly Gly Gly Gly Gly Gly Gly Gly Gly
        35                  40                  45

Gly

<210>  60
<211>  54
<212>  PRT
<213>  Artificial Sequence

<400>  60

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Gly
            20                  25                  30

Gly Cys Gly Gly Gly Gly Gly Gly Gly Gly Gly Gly Gly Gly Gly Gly
        35                  40                  45

Gly Gly Gly Gly Gly Gly
    50

<210>  61
<211>  47
<212>  PRT
<213>  Artificial Sequence

<400>  61

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Gly
            20                  25                  30

Gly Gly Gly Gly Cys Gly Gly Gly Gly Gly Gly Gly Gly Gly Gly
        35                  40                  45

<210>  62
<211>  52
<212>  PRT
<213>  Artificial Sequence

<400>  62

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Gly
            20                  25                  30

Gly Gly Gly Gly Cys Gly Gly Gly Gly Gly Gly Gly Gly Gly Gly Gly
        35                  40                  45

Gly Gly Gly Gly
    50

<210>  63
<211>  57
<212>  PRT
<213>  Artificial Sequence

<400>  63

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Gly
            20                  25                  30

Gly Gly Gly Gly Cys Gly Gly Gly Gly Gly Gly Gly Gly Gly Gly Gly
        35                  40                  45

Gly Gly Gly Gly Gly Gly Gly Gly Gly
    50                  55

<210>  64
<211>  43
<212>  PRT
<213>  Artificial Sequence

<400>  64

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Ala
            20                  25                  30

Cys Ala Ala Ala Ala Ala Ala Ala Ala Ala Ala
        35                  40

<210>  65
<211>  48
<212>  PRT
<213>  Artificial Sequence

<400>  65

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Ala
            20                  25                  30

Cys Ala Ala Ala Ala Ala Ala Ala Ala Ala Ala Ala Ala Ala Ala Ala
        35                  40                  45

<210>  66
<211>  53
<212>  PRT
<213>  Artificial Sequence

<400>  66

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Ala
            20                  25                  30

Cys Ala Ala Ala Ala Ala Ala Ala Ala Ala Ala Ala Ala Ala Ala Ala
        35                  40                  45

Ala Ala Ala Ala Ala
    50

<210>  67
<211>  44
<212>  PRT
<213>  Artificial Sequence

<400>  67

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Ala
            20                  25                  30

Ala Cys Ala Ala Ala Ala Ala Ala Ala Ala Ala Ala
        35                  40

<210>  68
<211>  49
<212>  PRT
<213>  Artificial Sequence

<400>  68

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Ala
            20                  25                  30

Ala Cys Ala Ala Ala Ala Ala Ala Ala Ala Ala Ala Ala Ala Ala Ala
        35                  40                  45

Ala

<210>  69
<211>  54
<212>  PRT
<213>  Artificial Sequence

<400>  69

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Ala
            20                  25                  30

Ala Cys Ala Ala Ala Ala Ala Ala Ala Ala Ala Ala Ala Ala Ala Ala
        35                  40                  45

Ala Ala Ala Ala Ala Ala
    50

<210>  70
<211>  47
<212>  PRT
<213>  Artificial Sequence

<400>  70

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Ala
            20                  25                  30

Ala Ala Ala Ala Cys Ala Ala Ala Ala Ala Ala Ala Ala Ala Ala
        35                  40                  45

<210>  71
<211>  52
<212>  PRT
<213>  Artificial Sequence

<400>  71

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Ala
            20                  25                  30

Ala Ala Ala Ala Cys Ala Ala Ala Ala Ala Ala Ala Ala Ala Ala Ala
        35                  40                  45

Ala Ala Ala Ala
    50

<210>  72
<211>  57
<212>  PRT
<213>  Artificial Sequence

<400>  72

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Ala
            20                  25                  30

Ala Ala Ala Ala Cys Ala Ala Ala Ala Ala Ala Ala Ala Ala Ala Ala
        35                  40                  45

Ala Ala Ala Ala Ala Ala Ala Ala Ala
    50                  55

<210>  73
<211>  43
<212>  PRT
<213>  Artificial Sequence

<400>  73

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Val
            20                  25                  30

Cys Val Val Val Val Val Val Val Val Val Val
        35                  40

<210>  74
<211>  48
<212>  PRT
<213>  Artificial Sequence

<400>  74

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Val
            20                  25                  30

Cys Val Val Val Val Val Val Val Val Val Val Val Val Val Val Val
        35                  40                  45

<210>  75
<211>  53
<212>  PRT
<213>  Artificial Sequence

<400>  75

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Val
            20                  25                  30

Cys Val Val Val Val Val Val Val Val Val Val Val Val Val Val Val
        35                  40                  45

Val Val Val Val Val
    50

<210>  76
<211>  44
<212>  PRT
<213>  Artificial Sequence

<400>  76

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Val
            20                  25                  30

Val Cys Val Val Val Val Val Val Val Val Val Val
        35                  40

<210>  77
<211>  49
<212>  PRT
<213>  Artificial Sequence

<400>  77

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Val
            20                  25                  30

Val Cys Val Val Val Val Val Val Val Val Val Val Val Val Val Val
        35                  40                  45

Val

<210>  78
<211>  54
<212>  PRT
<213>  Artificial Sequence

<400>  78

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Val
            20                  25                  30

Val Cys Val Val Val Val Val Val Val Val Val Val Val Val Val Val
        35                  40                  45

Val Val Val Val Val Val
    50

<210>  79
<211>  47
<212>  PRT
<213>  Artificial Sequence

<400>  79

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Val
            20                  25                  30

Val Val Val Val Cys Val Val Val Val Val Val Val Val Val Val
        35                  40                  45

<210>  80
<211>  52
<212>  PRT
<213>  Artificial Sequence

<400>  80

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Val
            20                  25                  30

Val Val Val Val Cys Val Val Val Val Val Val Val Val Val Val Val
        35                  40                  45

Val Val Val Val
    50

<210>  81
<211>  57
<212>  PRT
<213>  Artificial Sequence

<400>  81

His Ala Glu Gly Thr Phe Thr Ser Asp Val Ser Ser Tyr Leu Glu Gly
1               5                   10                  15

Gln Ala Ala Lys Glu Phe Ile Cys Trp Leu Cys Lys Gly Arg Gly Val
            20                  25                  30

Val Val Val Val Cys Val Val Val Val Val Val Val Val Val Val Val
        35                  40                  45

Val Val Val Val Val Val Val Val Val
    50                  55

<210>  82
<211>  52
<212>  PRT
<213>  Artificial Sequence

<400>  82

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly Gly
            20                  25                  30

Gly Gly Gly Gly Gly Gly Gly Gly Gly Cys Gly Gly Gly Gly Gly Gly
        35                  40                  45

Gly Gly Gly Gly
    50

<210>  83
<211>  47
<212>  PRT
<213>  Artificial Sequence

<400>  83

His Ala Glu Gly Thr Phe Thr Ser Asp Val Ser Ser Tyr Leu Glu Cys
1               5                   10                  15

Gln Ala Ala Lys Glu Phe Ile Ala Trp Leu Val Lys Gly Arg Gly Gly
            20                  25                  30

Gly Gly Gly Gly Cys Gly Gly Gly Gly Gly Gly Gly Gly Gly Gly
        35                  40                  45

<210>  84
<211>  55
<212>  PRT

<213>  Artificial Sequence

<400>  84

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly Gly
            20                  25                  30

Gly Gly Cys Gly Gly Gly Gly Gly Gly Gly Gly Gly Gly Gly Gly Gly
        35                  40                  45

Gly Gly Gly Gly Gly Gly Gly
    50                  55

<210>  85
<211>  31
<212>  PRT
<213>  Human

<400>  1

His Ala Glu Gly Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30